(19) Europäisches Patentamt
European Patent Office
Office européen des brevets

(11) **EP 3 802 868 B1**

(12) **EUROPEAN PATENT SPECIFICATION**

(45) Date of publication and mention
of the grant of the patent:
**11.03.2026 Bulletin 2026/11**

(21) Application number: **19729287.3**

(22) Date of filing: **07.06.2019**

(51) International Patent Classification (IPC):
*C12Q 1/6844* (2018.01)    *C12Q 1/6895* (2018.01)

(52) Cooperative Patent Classification (CPC):
(C-Sets available)
**C12Q 1/6895; C12Q 1/6844;** C12Q 2600/156
(Cont.)

(86) International application number:
**PCT/EP2019/065047**

(87) International publication number:
**WO 2019/234252 (12.12.2019 Gazette 2019/50)**

(54) **METHOD FOR DETECTING A TANDEM REPEAT**

VERFAHREN ZUR DETEKTION VON VARIABLEN SEQUENZWIEDERHOLUNGEN

PROCÉDÉ DE DÉTECTION D'UNE RÉPÉTITION EN TANDEM

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.06.2018 GB 201809450**

(43) Date of publication of application:
**14.04.2021 Bulletin 2021/15**

(73) Proprietor: **Imperial College Innovations Limited**
**London SW7 2AZ (GB)**

(72) Inventors:
• **GEORGIOU, Pantelis**
**London SW7 2AZ (GB)**
• **YU, Ling-Shan**
**London SW7 2AZ (GB)**
• **CARDENAS, Kenny, Malpartida**
**London SW7 2AZ (GB)**
• **FISHER, Matthew, C.**
**London SW7 2AZ (GB)**
• **MANZANO, Jesus, Rodriguez**
**London SW7 2AZ (GB)**
• **MOSER, Nicolas**
**London, SW7 2AZ (GB)**

(74) Representative: **Leonard, Thomas Charles et al**
**Kilburn & Strode LLP**
**Lacon London**
**84 Theobalds Road**
**London WC1X 8NL (GB)**

(56) References cited:
**WO-A1-2008/137715     WO-A1-2010/040374
CN-A- 105 624 290**

• **Y. KIMURA ET AL: "Optimization of turn-back
primers in isothermal amplification", NUCLEIC
ACIDS RESEARCH, vol. 39, no. 9, 1 May 2011
(2011-05-01), pages e59 - e59, XP055164865,
ISSN: 0305-1048, DOI: 10.1093/nar/gkr041**
• **JINZHAO SONG ET AL: "Molecular Detection of
Schistosome Infections with a Disposable
Microfluidic Cassette", PLOS NEGLECTED
TROPICAL DISEASES, vol. 9, no. 12, 31
December 2015 (2015-12-31), pages e0004318,
XP055567272, DOI: 10.1371/journal.pntd.0004318**
• **HAROLD SALANT ET AL: "The Development of a
Loop-Mediated Isothermal Amplification Method
(LAMP) for Echinococcus granulosis
Coprodetection", AMERICAN JOURNAL OF
TROPICAL MEDICINE & HYGIENE., vol. 87, no. 5,
7 November 2012 (2012-11-07), US, pages 883 -
887, XP055604673, ISSN: 0002-9637, DOI:
10.4269/ajtmh.2012.12-0184**

- **G. M. CHONG ET AL: "PCR-based detection of Aspergillus fumigatus Cyp51A mutations on bronchoalveolar lavage: a multicentre validation of the AsperGenius assay in 201 patients with haematological disease suspected for invasive aspergillosis", JOURNAL OF ANTIMICROBIAL CHEMOTHERAPY., vol. 71, no. 12, 15 August 2016 (2016-08-15), GB, pages 3528 - 3535, XP055617375, ISSN: 0305-7453, DOI: 10.1093/jac/dkw323**
- **CARLOS ALVAREZ-MORENO ET AL: "Azole-resistant Aspergillus fumigatus harboring TR34/L98H, TR46/Y121F/T289A and TR53 mutations related to flower fields in Colombia", SCIENTIFIC REPORTS, vol. 7, no. 1, 30 March 2017 (2017-03-30), XP055617344, DOI: 10.1038/srep45631**

Remarks:

The complete document including Reference Table(s) and the Sequence Listing(s) can be downloaded from the EPO website

(52) Cooperative Patent Classification (CPC): (Cont.)

C-Sets
**C12Q 1/6844, C12Q 2531/119**

**Description**

The present application relates to methods for detecting a tandem repeat in a nucleic acid sequence under isothermal conditions using primers.

The usefulness of the invention may be illustrated with respect to the fungus *Aspergillus fumigatus,* which is a globally occurring saprophyte and is highly prevalent in air owing to its production of numerous airborne conidia. Clinically, *A. fumigatus* causes a spectrum of respiratory and invasive infections, ranging from asthma-like symptoms to invasive aspergillosis in immunocompromised hosts. In the clinic, triazole antifungal drugs are widely used to control this fungal infection due to their high effectiveness in disrupting membrane structure and fungal cell transport. However, fungicides are also an essential means of controlling fungal disease in agriculture with sterol demethylation inhibitor (DMI) compounds, a class of chemicals which includes triazoles and imidazoles, representing the largest class of fungicides used worldwide. Environmentally, the evolution of widespread resistance to DMI's is observed not only in plant-infecting fungal pathogens but also in the saprophytic soil-dwelling *A. fumigatus.* This observation suggests that the deployment of triazoles in agriculture has led to selection for antifungal resistance not only in target crop pathogens but also those fungal species that co-occur in the environment and including those that can opportunistically infect humans.

The use of long term azole therapy to treat patients with clinical *A. fumigatus* infections is known to lead to the de novo evolution of resistance (Snelders et al., Future Microbiology, 6(3), 335-347, 2011). However, naïve patients who have not been previously treated with medical azoles are increasingly being diagnosed with multiazole resistant infections, leading to the hypothesis that these patients may have contracted infectious agents that have evolved resistance to azoles owing to their exposure to agricultural DMI's in the environment (Snelders et al., Applied and Environmental Microbiology, 75(12), 4053-4057, 2009). Azole resistance in *A. fumigatus* is associated with a tandem repeat within the promoter and mutations in the cyp51A gene, which encodes the drug target lanosterol 14-$\alpha$-demethylase. Specifically, an azole resistance mechanism characterised by a 34 bp tandem repeat (TR34) and a leucine-for-histidine substitution (L98H) in cyp51A is increasingly found to be present in patients manifesting infections with azole-resistant *A. fumigatus,* and in some recent studies over 90% of patients with triazole resistant *A. fumigatus* infections carry the TR34/L98H allele (Rivero-Menedez et al., Journal of Fungi, 2(3), 21, 2016; Verweij et al., Clinical Infectious Diseases, 62(3), 362-368, 2016). Importantly, *A. fumigatus* with this TR34/L98H combination has been also found widely in environmental samples that include soils, compost, plant bulbs and air (Rivero-Menedez *et al.,* 2016; Verweij *et al.,* 2016; Chowdhary et al., Journal of Antimicrobial Chemotherapy, 67(2), 362-366, 2013). Epidemiological studies using highly discriminatory genetic and genomic methods have shown the occurrence of closely related *A. fumigatus* genotypes containing the TR34/L98H allele both in nature and in clinical infections, strengthening the suspicion that azole-resistant isolates of this fungus in nature are causing drug resistant infection in the clinic as a consequence of the dual-use of DMI antifungals (Snelders et al., 2009). Consequently, there is an urgent need for rapid diagnostic methods targeted at detecting and managing multiazole resistant A. *fumigatus* both within the clinic and the field. Conventional diagnostic methods, such as culture, microscopic examination and radiology are commonly used for detecting infections caused by *A. fumigatus* (De Pauw et al., C. Clinical Infectious Diseases, 46(12), 1813-1821, 2008). However, these methods require several days in order to observe the fungus, have only moderate specificity and require further tests in order to detect azole-resistant alleles (Cuenca-Estrella et al., Journal of Antimicrobial Chemotherapy, 66 (SUPPL. 1). 2011). Antibody-based tests have been developed to rapidly detect infections, however are not able to determine the occurrence of azole-resistance (Miceli & Maertens, Seminars in Respiratory and Critical Care Medicine, 36(5), 650-661, 2015; White et al., Journal of Clinical Microbiology, 51(5), 1510-1516, 2013). PCR-based molecular methods have been developed due to their higher sensitives and specificities compared to conventional methods and are also able to detect the presence of azole-resistance alleles (Costa et al., Journal of Clinical Microbiology, 40(6), 2224-2227, 2002; Donnelly, Clinical Infectious Diseases, 42(4), 487-489, 2006; Kawazu et al., Journal of Clinical Microbiology, 42(6), 2733-2741, 2004; Loeffler et al., The Journal of Infectious Diseases, 185(8), 1203-1206, 2002; Marr & Leisenring, Clinical Infectious Diseases, 41(S6), S381-S386, 2005; Pickering et al., Journal of Clinical Microbiology, 43(12), 5957-5962, 2005; Raad et al., Cancer, 94(4), 1032-1036, 2002; White et al., Clinical Infectious Diseases, 61(8), 1293-1303, 2015). However, PCR-based detection requires laboratory equipment and experienced personnel. The continued increase of azole resistance, coupled with a lack of a rapid diagnostic method, raises the need for a simple, rapid, accurate diagnostic for antifungal mutations which is applicable in both field and clinical settings.

Loop-mediated isothermal amplification (LAMP) has been developed as a rapid, sensitive and simple technique for the detection of pathogens. This technique amplifies DNA in a highly specific, efficient and rapid manner while under isothermal conditions (Notomi et al., Nucleic Acids Research, 28(12), E63, 2000). The technique is based on the principle of auto-cycling and strand displacement DNA synthesis, using a DNA polymerase and a set of specially designed primers that recognise six distinct regions on a target DNA template. A further improvement to the speed of LAMP reaction is the introduction of two additional loop primers (Nagamine et al., Molecular and Cellular Probes, 16(3), 223-229, 2002). LAMP products can be visualised in different ways, including measuring turbidity, pH-sensitive dyes and metal indicators. Consequently, the technique has great potential for early diagnosis and may facilitate the development of point-of-care devices using lab on a chip technology. Jinzhao Song et al. (PLOS NEGLECTED TROPICAL DISEASES, 9(12), e0004318, 2015) and Harold Salant et al. (AMERICAN JOURNAL OF TROPICAL MEDICINE & HYGIENE., 87(5),883-887, 2012) describe the use of LAMP for the detection of tandem repeats.

Summary of the invention

The invention is defined by the claims. Any subject-matter falling outside the scope of the claims is provided for information purposes only.

Described herein is a new method for detection of tandem repeats. The new method may involve an application of LAMP chemistry which is particularly effective for the rapid detection of long and direct tandem repeats. The methodology described herein may be termed TR-LAMP and enables design of primers to detect tandem repeats more broadly.

The inventors have also designed specific LAMP primers targeting dominant triazole-resistant genotypes of *A. fumigatus* and demonstrate that the method disclosed herein has high analytical sensitivities and specificity for detecting the most prevalent cyp51A mutation, TR34. Owing to high reliability and rapid identification, the TR34-LAMP assay can be used as a novel diagnosis of invasive aspergillosis (IA) infection and may be coupled to point-of-care diagnostic platforms.

According to a first aspect, the invention provides a method for detecting a tandem repeat in a nucleic acid sequence.

According to a second aspect, the invention provides a method for detecting drug resistance in an organism comprising detecting a tandem repeat in a nucleic acid sequence.

According to a third aspect, the invention provides a method for the diagnosis of an infectious disease or a drug resistant infection comprising detecting a tandem repeat in a nucleic acid sequence.

According to a fourth aspect, a method for the treatment of an infectious disease or a drug resistant infection in a subject in need thereof comprising detecting a tandem repeat in a nucleic acid sequence is decribed herein (not claimed).

According to a fifth aspect, the invention provides a kit comprising primers used in the first to the fifth aspects of the invention.

According to a sixth aspect, the invention provides a reaction mixture comprising the components of the kit according to the sixth aspect of the invention in a liquid medium.

Reference is made to a number of Figures as follows:

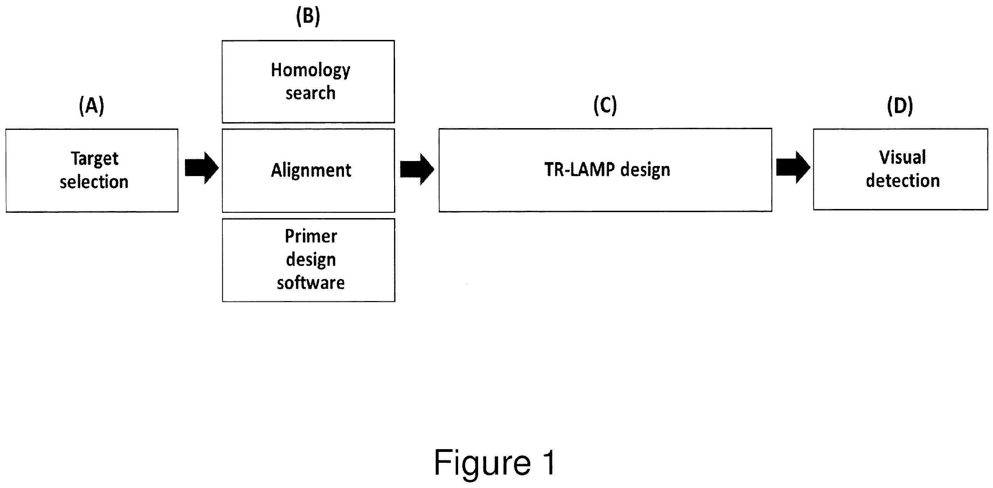

Figure 1 - The workflow used for TR-LAMP assay. (A) Select the drug-resistance related gene (B) Search for homology in NCBI nucleotide database and build the alignment. The alignment is used as a reference for primer design (LAMP primer design website: https://primerexplorer.jp/e/) (C) Manually design for FIP or BIP for a specific tandem repeat detection (D) Visual detection and threshold adjustment for a binary result.

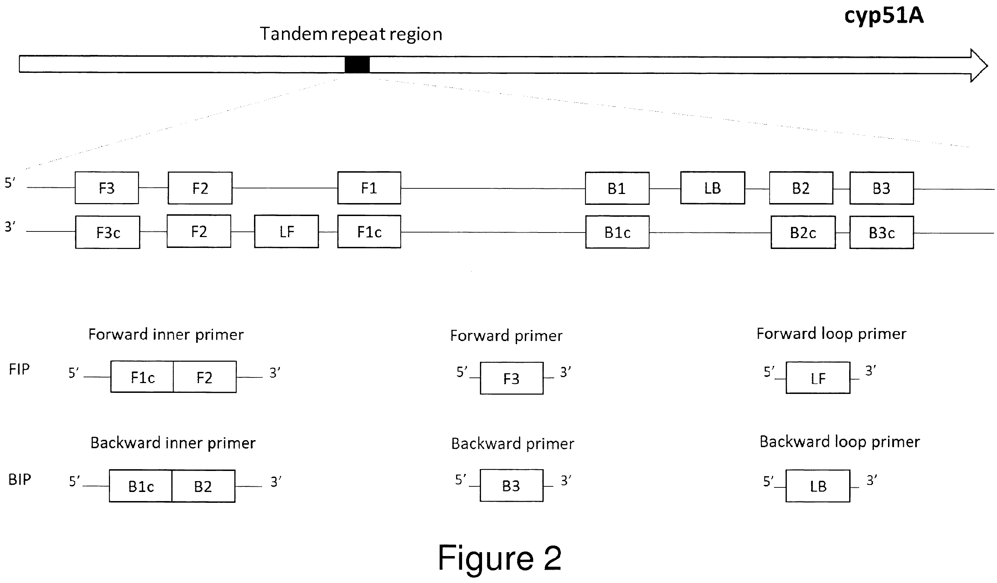

Figure 2 - A schematic diagram of *cyp51A* gene showing the position and composition of TR-LAMP primers for amplification of the tandem repeat region. Forward outer primer (F3), backward outer primer (B3), forward inner primer FIP (F1c+F2), backward inner primer BIP (B1c+B2), loop forward (LF) and loop backward (LB).

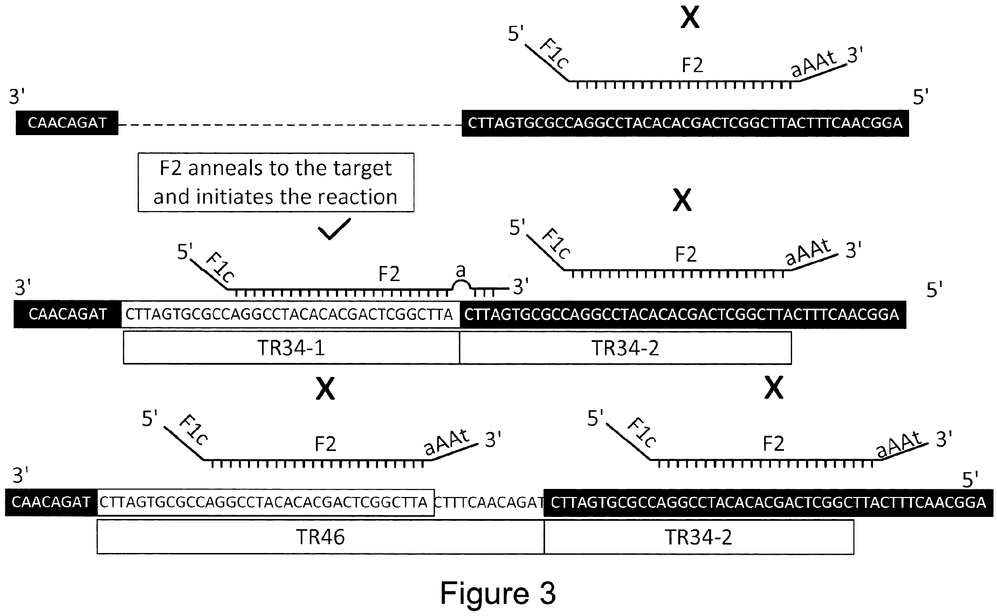

Figure 3 - A schematic figure of the detection for the TR34 tandem repeat regions by using the TR34-LAMP assay. Partial sequences of TR34 (EU626235.1), TR46 (MF070878.1) and wildtype (KJ210331.1) are shown. A labelled box indicates TR34 repeat unit 1 (TR34-1), another labelled box indicates TR34 repeat unit 2 (TR34-2) and another labelled box indicates TR46 repeat unit. The levels of similarity among three sequences were highlighted and categorised: highly conserved (black), conserved between mutants (grey) and presented only in one of the sequences (white). FIP primer (F1c + F2) used in TR34-LAMP assay is shown in black lines. Due to the introduced mismatches (nucleotides showed in lowercase) at 3' end of F2, we developed a one step yes/no reaction to differentiate TR34 from other strains by using one set of LAMP primers. The positive reaction (F2 attached to the target and initiate the reaction) is indicated by a tick, while the negative reactions are indicated by crosses. Vertical short black lines show that the primer has exact match with the template DNA.

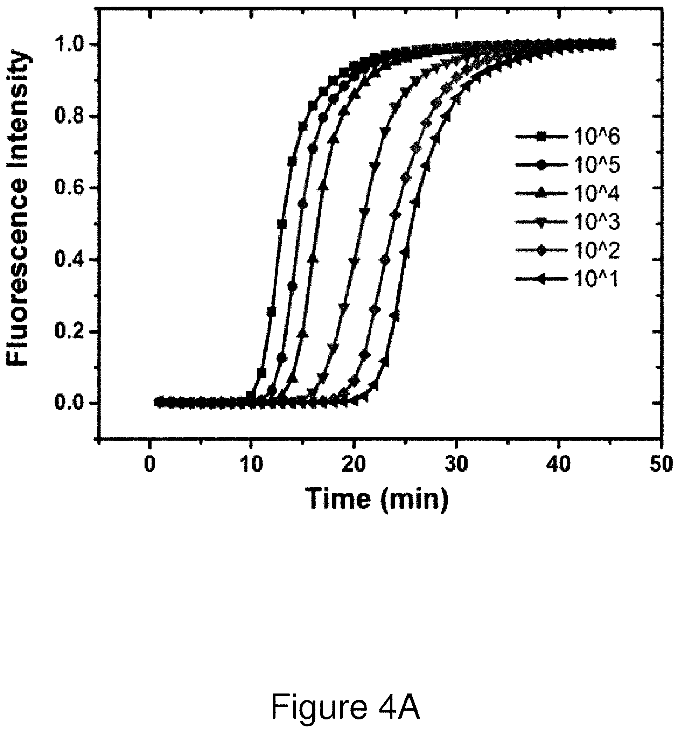

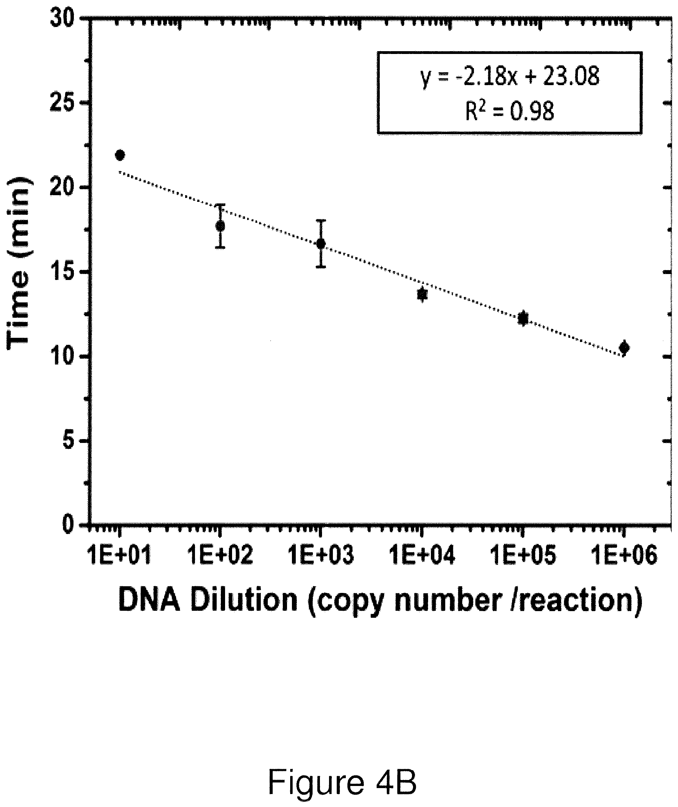

Figure 4 - Real-time TR34-LAMP assay amplification plots and standard curve. (a) Detection of different concentration of synthetic TR34 DNA. As low as 10 copies/reaction were detection within 22 min. (b) Standard curve for TR34-LAMP assay generated from the amplification plot between serial 10-fold dilutions of TR34 synthetic DNA. Three replicates per each concentration was used. Negative controls were included, which remain negative for 30 min. Error bars indicate standard deviation.

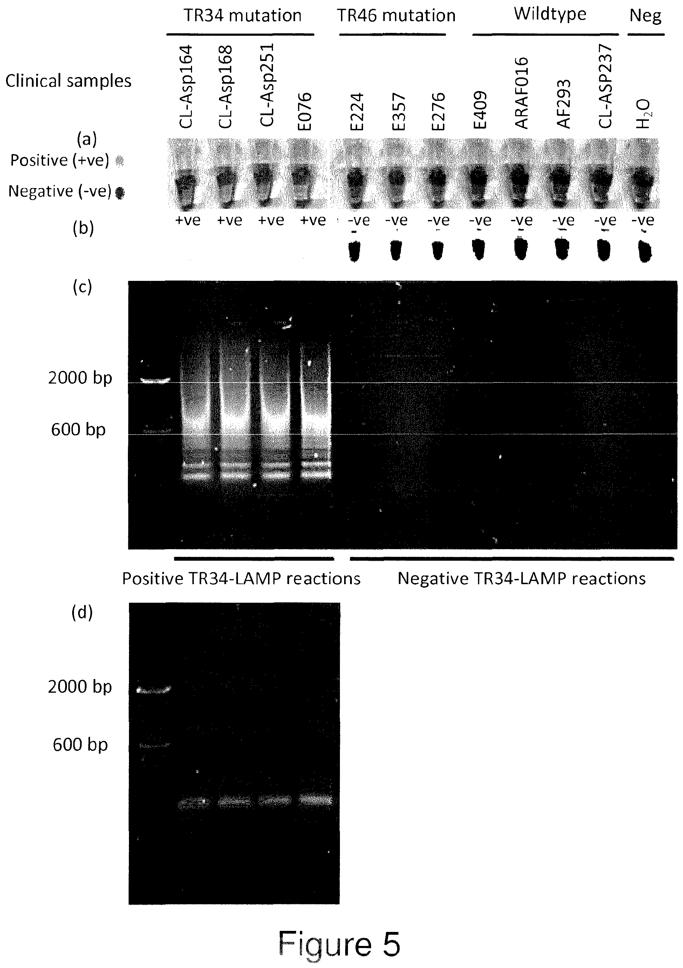

Figure 5 - Specificity the TR34-LAMP assay. (a) Visualisation of the TR34-LAMP products by colorimetric LAMP method. (b) Binary results obtained by adjusting the threshold of the photo taken by an iPhone 6 camera. (c) Assessment was based on gel electrophoresis analysis of TR34-LAMP products. (d) Restriction enzyme digestion of positive TR34-LAMP products.

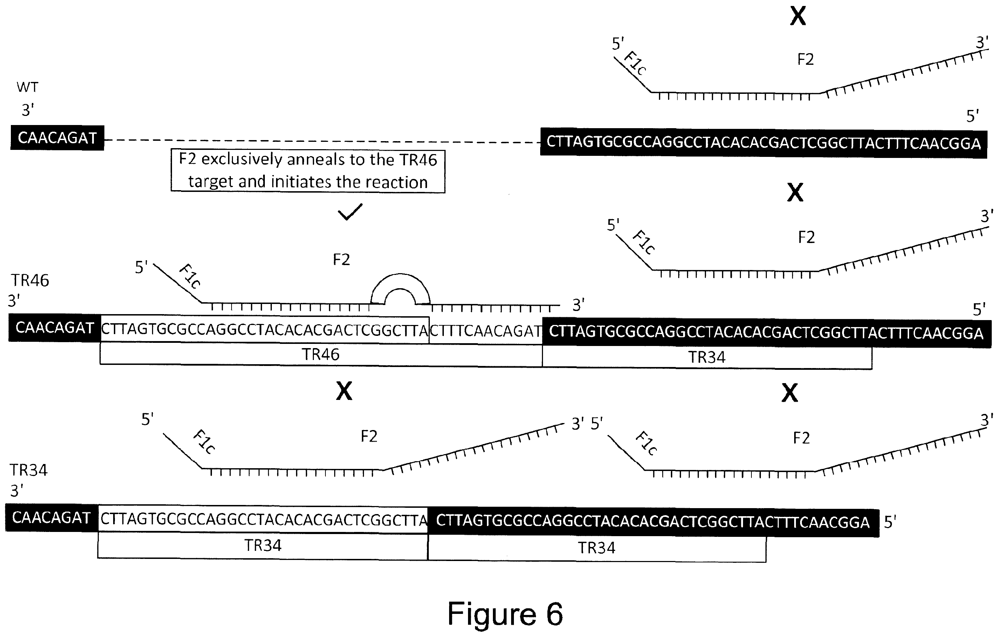

Figure 6 - A schematic figure of the detection for the TR46 tandem repeat regions. Partial sequences of TR34 (EU626235.1), TR46 (MF070878.1) and wildtype (KJ210331.1) are shown. A labelled box indicates TR34 repeat unit (TR34), another labelled box indicates TR46 repeat unit (TR46). The levels of similarity among three sequences were highlighted and categorised: highly conserved (black), conserved between mutants (grey), presented only in one of the sequences (white), gaps (dash lines). Due to the introduced mismatches at the misaligned region between TR46 and TR34, we developed a one step yes/no reaction to differentiate TR46 mutant from other strains by using one set of LAMP primers. The misaligned regions between template and primers are highlighted. The positive reaction (F2 attached to the target and initiate the reaction) is indicated by a tick, while the negative reactions are indicated by crosses. Vertical short black lines show that the primer has exact match with the template DNA.

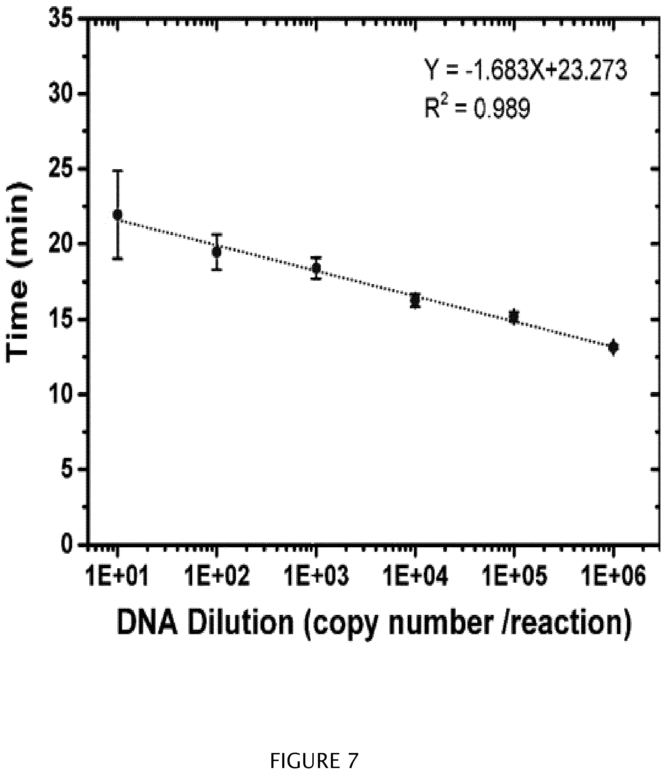

Figure 7 - Standard curve for TR46-LAMP assay generated from the amplification plot between serial 10-fold dilutions of TR46 DNA template. Three replicates per each concentration was used. Negative controls were included, which remain negative for 30 min. Error bars indicate standard deviation.

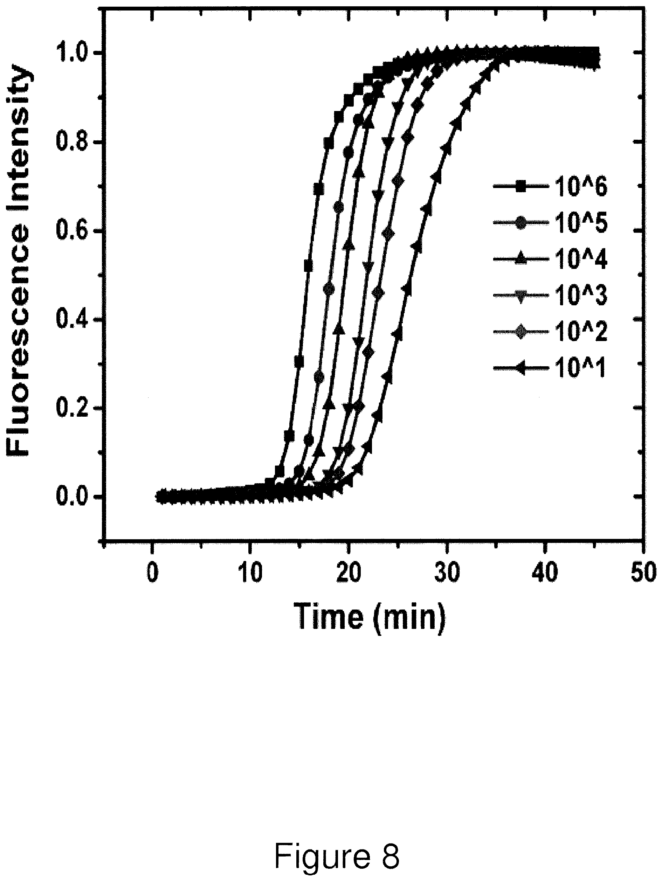

Figure 8 - Detection of different concentration of TR46 DNA template. As low as 10 copies/reaction were detection within 25 min.

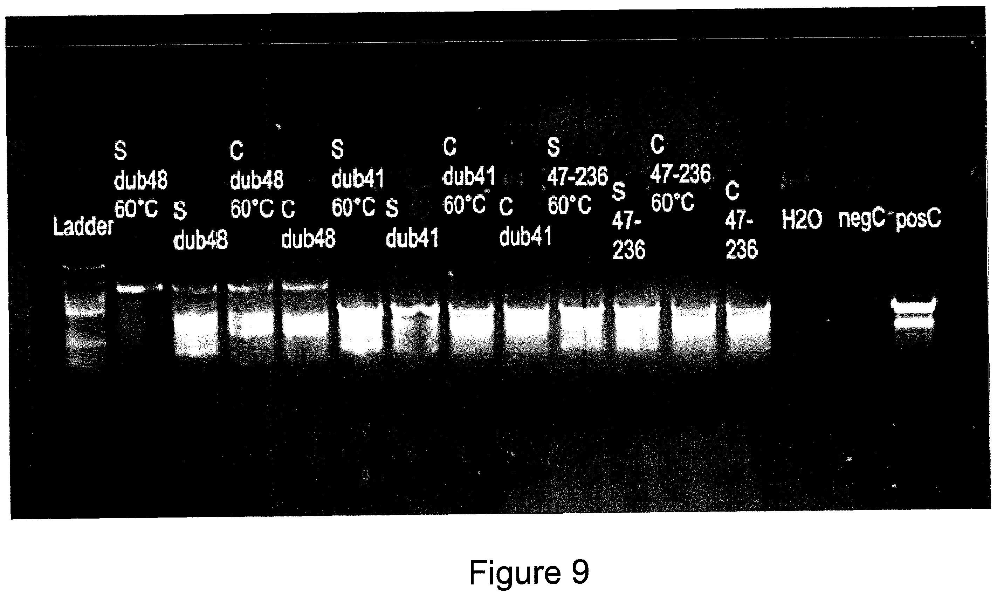

Figure 9 - Image visualising electrophoresis of MAT-type PCR products confirming the amplification for DUB41 (TR34), DUB48 (TR46) and 47-236 (WT) at different conditions (with or without 60°C incubation step). Negative control, water and positive control (known pure *A. fumigatus* gDNA) were also tested.

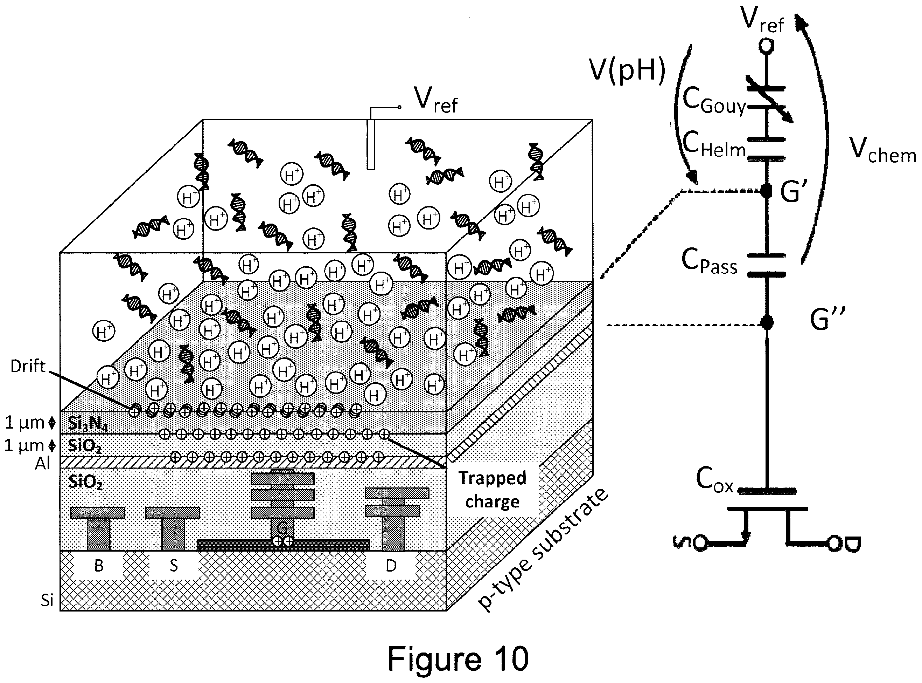

Figure 10 - Structure of the ISFET in unmodified CMOS technology and equivalent macromodel.

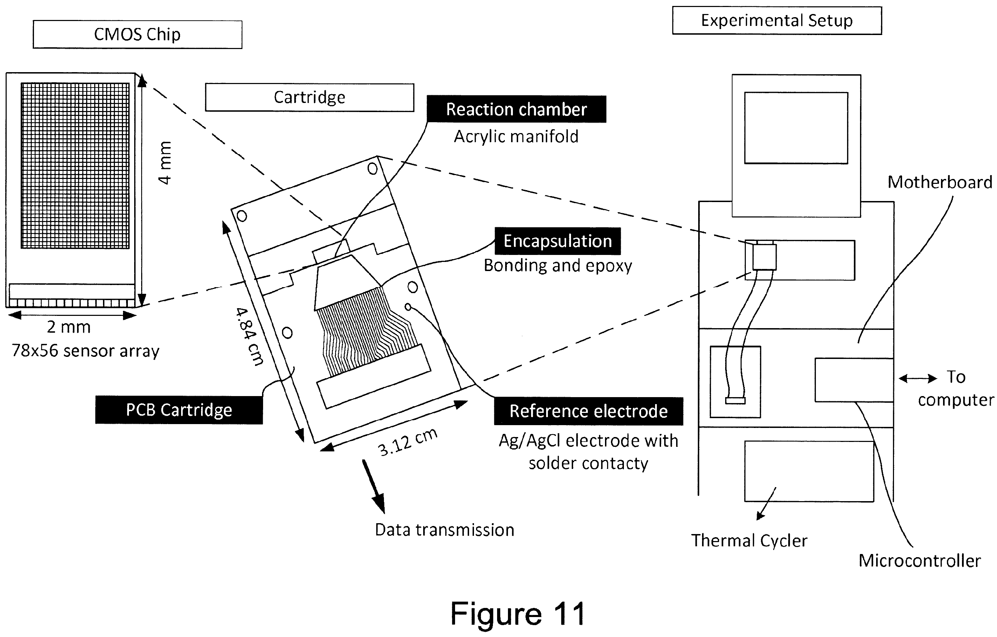

Figure 11 - Experimental setup for the nucleic acid amplification using the CMOS-based lab-on-a-chip platform. The chip integrating the 78x56 sensor array is mounted on a cartridge and an acrylic manifold is used as a reaction chamber

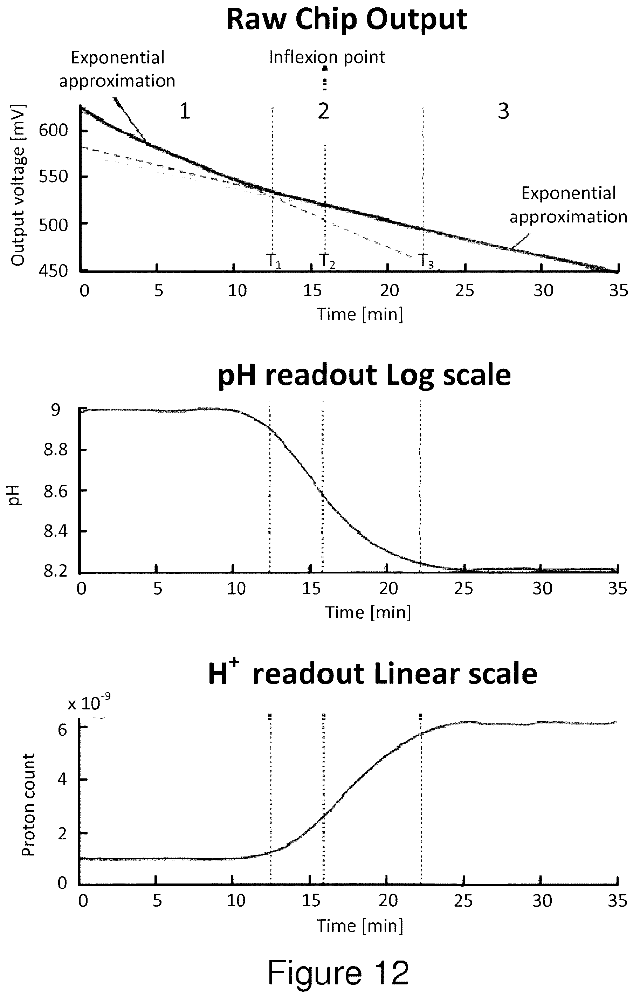

Figure 12 - Processing algorithm to extract the amplification curve from the averaged sensor outputs.

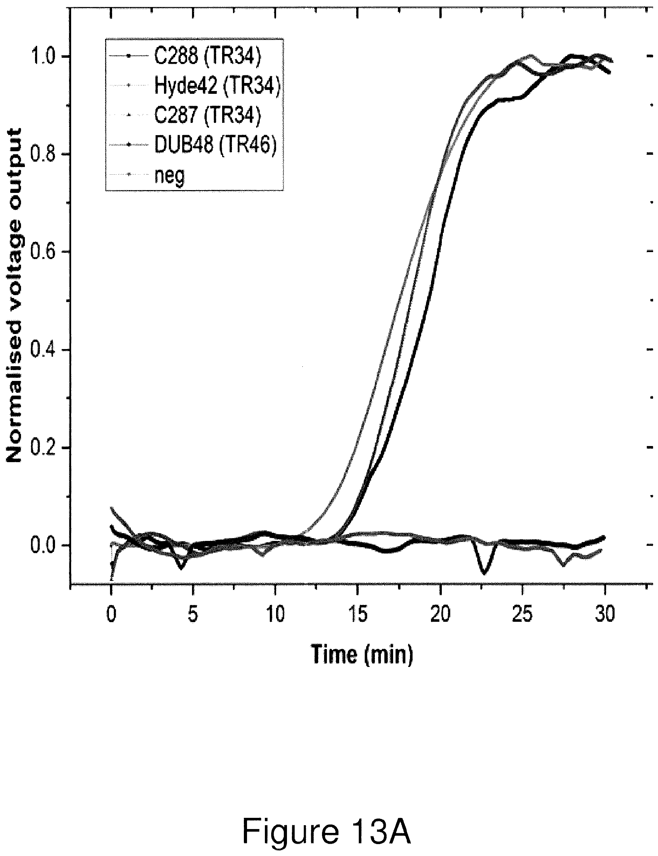

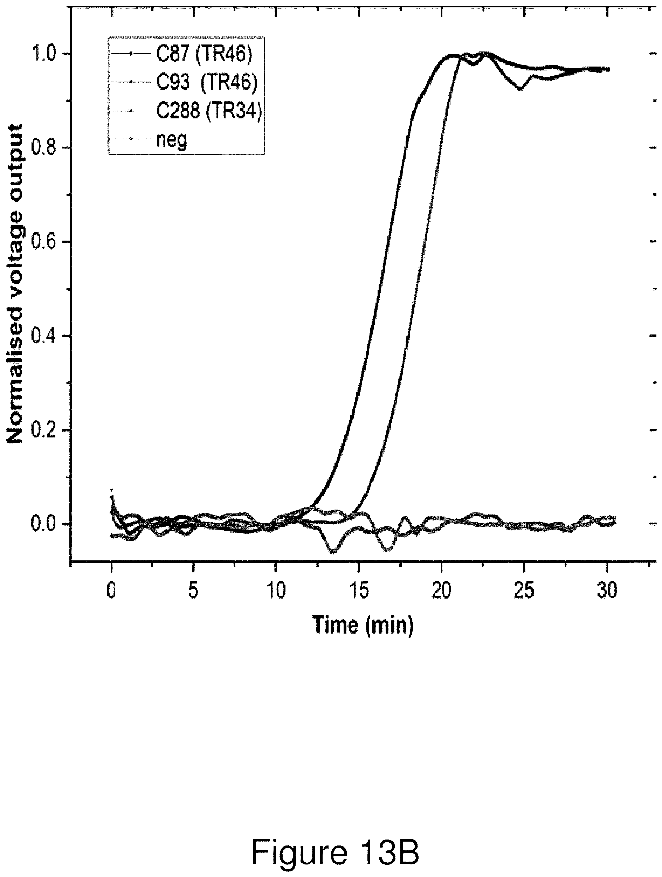

Figure 13 - On-chip amplification and detection using pH-sensitive TR-LAMP assays. Plotted data represent the signal obtained from the integrated circuit for the TR34 mutant samples (C287, C288 and Hyde42), TR46 mutant (C87 and C93) and non-template control (neg). (A) On-chip pH-sensitive TR34-LAMP amplification curves of TR34, TR46 mutants and non-template control. (B) On-chip pH-sensitive TR46-LAMP amplification curves of TR34, TR46 mutants and non-template control. The neg represent the non-template control.

<u>Detailed description of the invention</u>

According to a first aspect, the invention provides a method for detecting a tandem repeat in a nucleic acid sequence, wherein the tandem repeat comprises two or more repeat units each of which comprises at least two base pairs, wherein the method comprises

(a) amplifying under isothermal conditions and stringent conditions a nucleic acid sequence from a sample, in a reaction mixture comprising

    (i) the nucleic acid sequence,
    (ii) a nucleic acid polymerase,
    (iii) a nucleoside triphosphate mixture,
    (iv) a forward inner primer (FIP), and
    (v) a backward inner primer (BIP),

wherein the FIP or BIP anneals to a target region comprising one or more nucleotides from each of two repeat units, wherein the two repeat units are adjacent, and
(b) detecting an amplified nucleic acid sequence, wherein the detection of an amplified nucleic acid sequence indicates the presence the tandem repeat.

Nucleic acid amplification occurs under isothermal conditions. Accordingly, the method may also comprise a step of holding the reaction mixture under isothermal conditions. The isothermal conditions may be any suitable conditions for DNA amplification without the need for cycling between different temperatures for different steps in the amplification reaction, in contrast to traditional polymerase chain reaction (PCR). Isothermal conditions may be maintained using a laboratory water bath or heat block. The temperature selected may be from 30 to 95 °C. The temperature may be 50 to 70°C. The temperature may be around 50, 51, 52, 53, 54, 55, 56, 57, 58, 59, 60, 61, 62, 63, 64, 65, 66, 67, 68, 69 or 70 °C. The temperature may be 60 to 65°C. For example, the temperature may be around 63 °C.

The duration of the amplifying step may be around 1 to 150 minutes. The duration of the amplifying step may be around 5 to 60 minutes. The duration of the amplifying step may be around 20 to 40 minutes. The duration of the amplifying step may be around 20, 21, 22, 23, 24, 25, 26, 27, 28, 29, 30, 31, 32, 33, 34, 35, 36, 37, 38, 39 or 40 minutes. For example, the amplification step may be around 30 minutes. The amplification step may be at least around 30 minutes. The amplification step may be around 35 minutes

Any duration of the amplification step as defined above may be combined with any reaction temperature as defined above as long as the reaction conditions allow for amplification and an amplification product can be detected. The temperature and duration may be optimised by comparison with a positive and/or a negative control. For example, a reaction temperature and duration may be selected to allow for detectable amplification of product in a positive control and/or with the nucleic acid from the sample but not to allow for detectable amplification in another sample and/or a negative control.

The amplifying may comprise an annealing step and an extension step. Accordingly, the amplifying may comprise annealing FIP and BIP to the nucleic acid sequence and extending FIP and BIP to produce an amplified nucleic acid sequence. The amplifying may comprise an initiation phase comprising the annealing step and the extension step. The initiation phase may comprise the F2 region of FIP annealing to the F2c region of the nucleic acid sequence and the B2 region of BIP annealing to the B2c region of the nucleic acid sequence. The amplifying may also comprise annealing additional primers to the nucleic acid sequence as further described below.

The amplifying may be loop mediated isothermal amplification (LAMP). Since the method relates to a modified LAMP assay that detects tandem repeats, the method may be described as "tandem repeat LAMP" or TR-LAMP.

When the amplifying is LAMP, the temperature may be 50 to 70 °C. When the amplifying is LAMP, the temperature may be 60 to 65°C.

When the amplifying is LAMP, the amplifying step may be around 5 to 60 minutes.

When the nucleic acid sequence is RNA or cDNA, the amplifying may be reverse transcription loop mediated isothermal amplification (RT-LAMP). Since the method relates to a modified LAMP assay that detects tandem repeats, when the amplifying is RT-LAMP, the method may be described as "tandem repeat RT-LAMP" or TR-RT-LAMP.

Detecting may be by any suitable means. For example, amplification products may be detected by measuring turbidity, by using pH-sensitive dyes, using nucleic acid intercalating agents and by using metal indicators. The reaction mixture may comprise a detection agent. The detection agent may be a pH-sensitive dye or a metal indicator or a nucleic acid intercalating agent.

Detecting may be colorimetric detection. Colorimetric detection may be by the naked eye, or by use of a cell phone. Detecting may be colorimetric detection compatible with the naked eye or a cell phone. The cell phone may detect amplification using a camera and/or an app. The app may be an app designed for use with the method of the invention. The app may be supplied by a third party. The app may be software supplied with the cell phone. The app may be an image processing app. A phone or other suitable camera device may capture an image for detection. The image may then be processed on another device or on the same device.

Detecting may be pH-based detection. For example, the reaction mixture may comprise a pH-sensitive indicator, such as a dye. The pH sensitive indicator may be a phenol red pH indicator. The phenol red pH indicator may allow visual detection of the accumulation of protons during the amplification reaction, providing a change in solution colour from pink (negative) to yellow (positive).

Detecting may be by one or more ion sensors. For example, the detecting may be by one or more semiconductor-based ion sensors. Detection using semiconductor-based ion sensors may be particularly advantageous at the point-of-care. The use of ion sensors, in particular semiconductor-based ion sensors for example the ion-sensitive field-effect transistor (ISFET), provide a cheap and easy-to-use diagnostic platform for chemical sensing which is suitable for point-of-care applications. The ion-sensitive field-effect transistor (ISFET) is a chemically sensitive transistor. An IFSET can be used to measure ion concentrations in solution. An array of such sensors can create a platform for chemical sensing. The detecting may therefore be performed in accordance with a method of UK application no. 1809420.1 or International application no. PCT/GB2019/051597.

Detecting may be by one or more complementary metal-oxide-semiconductor (CMOS) based ion sensors. The detecting may therefore be by a CMOS based detection system. CMOS based detection systems employ label free amplification-coupled detection methods for measuring released hydrogen ions during nucleotide incorporation rather than relying on indirect measurements such as fluorescent dyes. CMOS technology therefore reduces the cost of the assay and size/price of instrumentation as there is no need for bulky equipment. Also, CMOS technology integrates thousands of sensors per microchips, enabling machine learning and analytical methods to reduce time-to-positive reaction, increasing sensitivity/specificity and enhancing quantification. These effects may be achieved in combination with a method of UK application no. 1809418.5 or International application no. PCT/EP2019/065039.

Relating to the field of Machine Learning, the method of UK application no. 1809418.5 or International application no. PCT/EP2019/065039 takes a multidimensional view, combining multiple features (e.g. linear features) in order to take advantage of, and improve on, information and principles behind existing methods to analyse real-time amplification data. The disclosed method involves two new concepts: the multidimensional standard curve and its 'home', the feature space. Together they expand the capabilities of standard curves, allowing for simultaneous absolute quantification, outlier detection and providing insights into amplification kinetics. This disclosure describes a general method which, for the first time, presents a multi-dimensional standard curve, increasing the degrees of freedom in data analysis and thereby being capable of uncovering trends and patterns in real-time amplification data obtained by existing qPCR instruments (such as the LightCycler 96 System from Roche Life Science). It is believed that this disclosure redefines the foundations of analysing real-time nucleic acid amplification data and enables new applications in the field of nucleic acid research.

A method for use in quantifying a sample comprising a target nucleic acid is provided, the method comprising: obtaining a set of first real-time amplification data for each of a plurality of target concentrations; extracting a plurality of N features from the set of first data, wherein each feature relates the set of first data to the concentration of the target; and fitting a line to a plurality of points defined in an N-dimensional space by the features, each point relating to one of the plurality of target concentrations, wherein the line defines a multidimensional standard curve specific to the nucleic acid target which can be used for quantification of target concentration.

Optionally the method further comprises: obtaining second real-time amplification data relating to an unknown sample; extracting a corresponding plurality of N features from the second data; and calculating a distance measure between the line in N-dimensional space and a point defined in N-dimensional space by the corresponding plurality of N features. Optionally, the method further comprises computing a similarity measure between amplification curves from the distance measure, which can optionally be used to identify outliers or classify targets. Optionally each feature is different to each of the other features, and optionally wherein each feature is linearly related to the concentration of the target, and optionally wherein one or more of the features comprises one of $C_t$, $C_y$ and $-\log_{10}(F_0)$. Optionally the method further comprises mapping the line in N-dimensional space to a unidimensional function, $M_0$, which is related to target concentration, and optionally wherein the unidimensional function is linearly related to target concentration, and/or optionally wherein the unidimensional function defines a standard curve for quantifying target concentration. Optionally, the mapping is performed using a dimensionality reduction technique, and optionally wherein the dimensionality reduction technique comprises at least one of: principal component analysis; random sample consensus; partial-least squares regression; and projecting onto a single feature. Optionally, the mapping comprises applying a respective scalar feature weight to each of the features, and optionally wherein the respective feature weights are determined by an optimisation algorithm which optimises an objective function, and optionally wherein the objective function is arranged for optimisation of quantisation performance. Optionally, calculating the distance measure comprises projecting the point in N-dimensional space onto a plane which is normal to the line in N-dimensional space, and optionally wherein calculating the distance measure further comprises calculating, based on the projected point, a Euclidean distance and/or a Mahalanobis distance. Optionally, the method further comprises calculating a similarity measure based on the distance measure, and optionally wherein calculating a similarity measure comprises applying a threshold to the similarity measure. Optionally, the method further comprises determining whether the point in N-dimensional space is an inlier or an outlier based on the similarity measure. Optionally, the method further comprises: if the point in N-dimensional space is determined to be an outlier then excluding the point from training data upon which the step of fitting a line to a plurality of points defined in N-dimensional space is based, and if the point in N-dimensional space is not determined to be an outlier then re-fitting the line in N-dimensional space based additionally on the point in N-dimensional space. Optionally, the method further comprises determining a target concentration based on the multidimensional standard curve, and optionally further based on the distance measure, and optionally based on the unidimensional function which defines the standard curve. Optionally, the method further includes displaying the target concentration on a display. Optionally, the method further comprises a step of fitting a curve to the set of first data, wherein the feature extraction is based on the curve-fitted first data, and optionally wherein the curve fitting is performed using one or more of a 5-parameter sigmoid, an exponential model, and linear interpolation. Optionally, the set of first data relating to the melting temperatures is pre-processed, and the curve fitting is carried out on the processed set of first data, and optionally wherein the pre-processing comprises one or more of: subtracting a baseline; and normalisation. Optionally, the data relating to the melting temperature is derived from one or more physical measurements taken versus sample temperature, and optionally wherein the one or more physical measurements comprise fluorescence readings.

Detecting may therefore be a method for detecting an amplification reaction in a biological sample using an array of ion sensors, the amplification reaction being indicative of the presence of a pathogen. The method may comprise monitoring a signal from each respective sensor of the array of ion sensors. The method may further comprise detecting a change in the signal from a first sensor of the array of ion sensors. The method may further comprise comparing the signal from the first sensor with the signal of at least one neighbouring sensor, the neighbouring sensor being adjacent to the first sensor in the array. Even further, the method may comprise determining, based on the comparing, that an amplification event has occurred in the vicinity of the first sensor.

Detecting may use a nucleic acid intercalating agent. The nucleic acid intercalating agent may be a fluorescent intercalating agent. The fluorescent intercalating agent may, for example, be a cyanine dye such as SYBR® Green (IUPAC ID: N',N'-dimethyl-N-[4-[(E)-(3-methyl-1,3-benzothiazol-2-ylidene)methyl]-1-phenylquinolin-1-ium-2-yl]-N-propylpropane-1,3-diamine), EvaGreen® (CAS number:913830-62-3), SYTO™-9 , SYTO™-13, SYTO™-82 and SYBR® Gold, SYTO™-9 (CBN number:CB6328526) , SYTO™-13 (CAS number:173080-69-8), SYTO™-82 and SYBR® Gold (CAS number:163795-75-3).

Detecting may be detecting using a metal indicator. The metal indicator may, for example, be hydroxynaphthol blue, eriochrome black t, calmagite, curcumin, fast sulphon black, hematoxylin, murexide or xylenon orange.

Detecting may be performed in real time or at an end-point. Detecting in real time may be for example by using the LC96 System (LightCycler® 96 System, Roche, Switzerland). End-point detection may be, for example by using an end-point PCR machine, such as the VeritiTM Dx 96-well Fast Thermal Cycler (Thermo Fisher Scientific, USA), following by the colorimetric or fluorescent detection.

Detecting may be performed with reference to or by comparison with one or more controls. The control may be a positive and/or a negative control. Detecting may be performed with reference to or by comparison with a positive and/or a negative control. No amplification may be detected in the negative control. Amplification may be detected in the positive control. In the presence of a positive and/or negative control, the reaction mixture may be referred to as the test reaction mixture. Detecting may be performed by the finding of a significant difference in amplification compared to a positive and/or a negative control. The significant difference in amplification may be a significant difference in the time taken for an amplification product to be detected. Alternatively, the significant difference in amplification may be a significant difference in the time taken for a threshold level of an amplification product to be detected. The threshold level may be any suitable threshold level to permit a finding of a significant difference in amplification. The significant difference in amplification may be a significant difference in colour of the reaction mixture after a defined time.

The negative control may use a reaction mixture corresponding to the test reaction mixture except for the absence of the nucleic acid sequence. Alternatively, the negative control may use a reaction mixture corresponding to the test reaction mixture except the nucleic acid sequence is replaced with a different nucleic acid sequence known not to be amplified by the primers in the reaction mixture; in this situation, the nucleic acid sequence in the test reaction mixture may be termed the test nucleic acid sequence and the nucleic acid sequence in the negative control reaction mixture may be termed the negative control nucleic acid sequence.

The positive control may use a reaction mixture corresponding to the test reaction mixture except the nucleic acid sequence is replaced with a different nucleic acid sequence known to be amplified by the primers in the reaction mixture; in this situation, the nucleic acid sequence in the test reaction mixture may be termed the test nucleic acid sequence and the nucleic acid sequence in the positive control reaction mixture may be termed the positive control nucleic acid sequence. The positive control nucleic acid sequence may for example be a chemically synthesised nucleic acid sequence comprising the tandem repeat.

In an alternative definition, the detection step may not involve the detection of an amplified nucleic acid sequence indicates the presence the tandem repeat. Accordingly, step (b) of the method above may alternatively be stated as: (b) detecting an amplified nucleic acid sequence.

The nucleic acid sequence may be DNA or RNA. When the nucleic acid sequence is DNA, the nucleic acid polymerase is a DNA polymerase, Bst 2.0 DNA Polymerase, Bst 3.0 DNA Polymerase or Large fragment Bst DNA polymerase. Accordingly, when the nucleic acid polymerase is a DNA polymerase, the nucleoside triphosphate mixture comprises dNTPs. dNTPs comprise dATP, dGTP, dCTP, and dTTP.

When the nucleic acid sequence is RNA the method may also comprise reverse transcription of the RNA to produce cDNA, for example by addition of a reverse transcriptase such as AMV Reverse Transcriptase. The nucleic acid sequence may therefore also be cDNA. When the nucleic acid is cDNA, the nucleic acid polymerase is a DNA polymerase and the nucleoside triphosphate mixture comprises dNTPs.

The reaction mixture may be any suitable medium in which amplification of a nucleic acid may take place. The reaction mixture may be a liquid medium. The reaction mixture may comprise deionised water. The reaction medium may comprise isothermal buffer, MgSO$_4$, BSA, Betaine and/or Syto9. The reaction mixture may be of any suitable volume.

The reaction mixture may be in a vessel. The vessel may be referred to as a reaction vessel. The vessel may be any suitable container. The vessel may be open topped. For example, the vessel may be a test tube or a plate, such as a 96-well plate. The vessel may be closed, for example by the addition of a lid. The lid may be integral or separate. For example, the vessel may be a microcentrifuge tube such as an Eppendorf tube. The vessel may be of any suitable volume.

The optimal volume of reaction mixture may be determined by the skilled person, accounting for example for the size of the vessel. For instance, when the vessel is a 96-well plate the reaction volume may be from 5 to 50 μL. For example, the reaction volume may be 5 μL.

The method of the invention may be performed in a number of repeats. For example, the method may be performed in duplicate or triplicate.

As used herein, "stringent conditions" are known to those skilled in the art and can be found in Current Protocols in Molecular Biology, John Wiley & Sons, N.Y., 6.3.1-6.3.6, 1991. The stringent conditions may be stringent conditions for hybridisation. The stringent conditions may be stringent conditions for annealing. Stringent conditions may be defined as equivalent to annealing in isothermal buffer. Isothermal buffer (1x) may comprise 20 mM Tris-HCl; 10 mM $(NH_4)_2SO_4$; 50 mM KCl; 8 mM $MgSO_4$; Betaine 0.8M; 0.1% Tween® 20; and may have pH 8.8 at 25 °C.

As used herein, "nucleic acid sequence" may refer to either a double stranded or to a single stranded nucleic acid molecule. The nucleic acid sequence may therefore alternatively be defined as a nucleic acid molecule. The nucleic acid molecule comprises two or more nucleotides. The nucleic acid sequence may be synthetic. The nucleic acid sequence may refer to a nucleic acid sequence that was present in the sample on collection. Alternatively, the nucleic acid sequence may be an amplified nucleic acid sequence or an intermediate in the amplification of a nucleic acid sequence, such as a dumbbell shaped intermediate or a stem-loop intermediate, which are described further below.

As used herein, "anneal", "annealing", "hybridise" and "hybridising" refer to complementary sequences of single-stranded regions of a nucleic acid pairing *via* hydrogen bonds to form a double-stranded polynucleotide. As used herein, "anneal", "anneals", "hybridise" and "hybridises" may refer to an active step. Alternatively, as used herein, "anneal", "anneals", "hybridise" and "hybridises" may refer to a capacity to anneal or hybridise; for example, that a primer is configured to anneal or hybridise and/or that the primer is complementary to a target. Accordingly, for example, a reference to a primer or a region of a primer which anneals to a nucleic acid sequence or a region of a nucleic acid sequence may in a method of the invention mean either that the annealing is a required step of the method; that the primer or region of the primer is complementary to the nucleic acid sequence or region of the nucleic acid sequence; or that the primer or region of the primer is configured to anneal to the nucleic acid sequence or region of the nucleic acid sequence.

The term "primer" as used herein refers to a nucleic acid, whether occurring naturally as in a purified restriction digest or produced synthetically, which is capable of acting as a point of initiation of synthesis when placed under conditions in which synthesis of a primer extension product, which is complementary to a nucleic acid strand, is induced, i.e. in the presence of nucleotides and an inducing agent such as a DNA polymerase and at a suitable temperature and pH. The primer may be either single-stranded or double-stranded and must be sufficiently long to prime the synthesis of the desired extension product in the presence of the inducing agent. The exact length of the primer will depend upon many factors, including temperature, source of primer and the method used. For example, for diagnostic applications, depending on the complexity of the target sequence, the nucleic acid primer typically contains 15 to 25 or more nucleotides, although it may contain fewer or more nucleotides. According to the present invention a nucleic acid primer typically contains 13 to 30 or more nucleotides.

Two primers that anneal to opposite strands of a template nucleic acid molecule and may each initiate synthesis of the nucleic acid sequence between their two target sites may be referred to as a primer set. FIP and BIP may be referred to as a primer set since they may initiate synthesis of the nucleic acid sequence between F1/F1c and B1/B1c.

Since DNA synthesis occurs in the 5' to 3' direction, the 3' ends of a primer set point towards each other, when they are annealed to their template nucleic acid strand, and the primers anneal on opposite strands of the template nucleic acid strand. For example, the BIP may anneal to the template (-) strand and is identical to (a part of) the template (+) strand. Likewise, the FIP may anneal to the template (+) strand and is identical to (a part of) the template (-) strand. Forward, reverse, (+) and (-) refer to transcription of genes; the (+) DNA strand has the same orientation as a messenger RNA, transcribed from the DNA. Alternatively stated, the forward orientation is pointing towards the end of a gene.

FIP may comprise an F1c region and an F2 region. The FIP may consist of an F1c region and an F2 region. The F2 region may be displaced in the 3' direction with respect to the F1c region within FIP. The F2 region of FIP may comprise a nucleic acid that is complementary to the tandem repeat. The F2 region of FIP may be complementary to a target region. The target region may therefore be termed F2c. Alternatively, when the B2 region of BIP is complementary to the target region, the F2 region of FIP may not be complementary to the target region.

The F1c region of FIP is not complementary to the target region. Accordingly, the F1c region of FIP will not anneal to the target region under stringent conditions. Rather, the F1c region of FIP comprises a sequence that is the reverse complement of an F1 region of the nucleic acid. The F1 region of the nucleic acid is displaced from the F2 region of the nucleic acid in the 3' direction. The F1c region of FIP will therefore anneal to the F1 region of the nucleic acid under stringent conditions.

FIP may incorporate one or more mismatched nucleotides. The presence or absence of the one or more mismatched nucleotides is determined with respect to the sequence of the tandem repeat to which FIP anneals under stringent conditions. The one or more mismatched nucleotides may enhance the specificity of binding to the target region. The specificity of binding to the target region may be enhanced with respect to off-target binding to the end of the second repeat unit and/or the end of the tandem repeat.

Accordingly, the F2 region of FIP may be 100% complementary to the target region. If one or more mismatches are incorporated, then the F2 region of FIP may be less than 100% complementary to the target region. For example, the F2 region of FIP may be from 99% to 60% complementary to the target region. The F2 region of FIP may be from 99% to 80% complementary to the target region. The F2 region of FIP may be from 99% to 85% complementary to the target region. The F2 region of FIP may be from 99% to 90% complementary to the target region. The F2 region of FIP may be from 98% to 92% complementary to the target region. The F2 region of FIP may be from 96% to 94% complementary to the target region. The F2 region of FIP may be about 99%, 98%, 97%, 96%, 95%, 94%, 93%, 92%, 91% or 90% complementary to the target region.

Where more than one mismatched nucleotide is incorporated into the F2 region of FIP, the mismatched nucleotides may be sequential or non-sequential. The mismatched nucleotides may be equal in number to the corresponding nucleotides in the sequence of F2c. Alternatively, the mismatched nucleotides may be greater in number or fewer in number than the corresponding nucleotides in the sequence of F2c. The inventors have determined that an unequal number of nucleotides affected the specificity of both control and test reactions and affected the delay (or fail) to amplify the target.

The one or more mismatched nucleotides may be in the F2 region of FIP. The one or more mismatched nucleotides may be located proximal to the 3' end of FIP and/or the 3' end of the F2 region. Accordingly, the mismatches may be termed "3' mismatches". Proximal to the 3' end may be at any point closer to the 3' end of F2 than the 5' end of F2. For example, the one or more mismatched nucleotides may be in the 3' half of F2, the 3' third of F2, the 3' quarter of F2, the 3' fifth of F2, the 3' sixth of F2, the 3' seventh of F2, the 3' eighth of F2, the 3' ninth of F2 or the 3' tenth of F2. Similarly, proximal to the 3' end may be at any point closer to the 3' end of FIP than the 5' end of FIP. For example, the one or more mismatched nucleotides may be in the 3' half of FIP, the 3' third of FIP, the 3' quarter of FIP, the 3' fifth of FIP, the 3' sixth of FIP, the 3'seveth of FIP, the 3' eighth of FIP, the 3' ninth of FIP or the 3' tenth of FIP.

BIP may comprise an B1c region and an B2 region. BIP may consist of a B1c region and a B2 region. The B2 region of BIP may be displaced in the 3' direction with respect to the B1c region within BIP. The B2 region of BIP may comprise a nucleic acid that is complementary to the tandem repeat. The B2 region of BIP may be complementary to a target region. The target region may therefore be termed B2c. Alternatively, when the F2 region of FIP is complementary to the target region, the B2 region of BIP may not be complementary to the target region.

The B1c region of BIP is not complementary to the target region. Accordingly, the B1c region of BIP will not anneal to the target region under stringent conditions. Rather, the B1c region of BIP comprises a sequence that is the reverse complement of an B1 region of the nucleic acid. The B1 region of the nucleic acid is displaced from the B2 region of the nucleic acid in the 3' direction. The B1c region of BIP will therefore anneal to the B1 region of the nucleic acid under stringent conditions.

Alternatively, BIP, rather than FIP, may anneal to the target region comprising one or more nucleotides from each of two repeat units. Therefore, the features and effects described above in connection with FIP may be possessed by BIP *mutatis mutandis.*

For example, BIP may comprises a B1c region and a B2 region. The B2 region of BIP may anneals to the target region. The B2 region of BIP may comprise one or more mismatched nucleotides with respect to the target region. The one or more mismatched nucleotides may be located proximal to the 3' end of the B2 region of BIP. The B2 region of BIP may comprise 18 to 30 nucleotides.

The amplification may comprise formation of a "dumbbell shape" intermediate. The dumbbell shape intermediate is a nucleic acid sequence. The dumbbell shape intermediate may be a single nucleic acid molecule. The term "dumbbell shape" refers to a possible secondary structure of the intermediate. The single nucleic acid molecule may comprise one or more stem-loop structures.

The dumbbell shape intermediate may comprise, in the 5' to 3' direction a B1c region, a B2 region, a LBc region, a B1 region, a F1c region, a LF region, a F2c region and a F1 region. Since the B1c region and B1 region are complementary they may form a stem-loop structure, wherein the B1c and B1 regions anneal to form the stem and the B2 and LBc regions form the loop. The B2 and LBc regions forming the loop are single stranded. Likewise, Since the F1c region and F1 region are complementary they may form a stem-loop structure, wherein the F1c and F1 regions anneal to form the stem and the F2 and LF regions form the loop. The F2 and LF regions forming the loop are single stranded.

Similarly, the dumbbell shape intermediate may comprise, in the 5' to 3' direction a F1c region, a F2 region, a LFc region, a F1 region, a B1c region, a LB region, a B2c region and a B1 region. Since the B1c region and B1 region are complementary they may form a stem-loop structure, wherein the B1c and B1 regions anneal to form the stem and the B2 and LBc regions form the loop. The B2 and LBc regions forming the loop are single stranded. Likewise, Since the F1c region and F1 region are complementary they may form a stem-loop structure, wherein the F1c and F1 regions anneal to form the stem and the F2 and LF regions form the loop. The F2 and LF regions forming the loop are single stranded.

The amplification may further comprise formation of a stem-loop intermediate. The stem-loop intermediate is a nucleic acid sequence. The stem-loop intermediate may be a single nucleic acid molecule. The term "stem-loop" refers to a possible secondary structure of the intermediate. The single nucleic acid molecule may comprise one or more stem-loop structures.

The stem-loop intermediate may comprise, in the 5' to 3' direction a B1c region, a B2 region, a LBc region, a B1 region, a F1c region, a LF region, a F2c region and a F1 region. Since the F1c region and F1 region are complementary they may form a stem-loop structure, wherein the F1c and F1 regions anneal to form part of the stem and the F2 region forms the loop. The F2 and LF regions forming the loop are single stranded. Since the B1c region and B1 region are complementary they may form a stem-loop structure, wherein the B1c and B1 regions anneal to form part of the stem and the B2 and LBc regions forms the loop. The B2 and LBc regions forming the loop are single stranded.

Similarly, the stem-loop intermediate may comprise, in the 5' to 3' direction a F1c region, a F2 region, a LFc region, a F1 region, a B1c region, a LB region, a B2c region and a B1 region. Since the B1c region and B1 region are complementary they may form a stem-loop structure, wherein the B1c and B1 regions anneal to form part of the stem and the B2 region forms the loop. The B2 and BF regions forming the loop are single stranded. Since the F1c region and F1 region are complementary they may form a stem-loop structure, wherein the F1c and F1 regions anneal to form part of the stem and the F2 and LFc regions forms the loop. The F2 and LFc regions forming the loop are single stranded.

Without being bound by theory, the stem-loop intermediate may be formed by the addition of nucleotides to the 3' end of the dumbbell shape intermediate. The dumbbell shape intermediate may therefore be extended from the 3' end. Extension from the 3' end of the dumbbell shape intermediate opens up the loop at the 5' end of the dumbbell shape structure, thereby converting the dumbbell shape intermediate into a stem-loop intermediate.

The amplification may further comprise LAMP cycling. The LAMP cycling may be initiated by FIP and optionally LB hybridising to the loop of the stem-loop intermediate. The F2 region of FIP may hybridise to the F2c region of the loop of the stem-loop intermediate and optionally LB may hybridise to the LBc region of the loop of the stem-loop intermediate. Extension from the F2 region of FIP may displace the 3' end of the stem-loop intermediate (a new B1c and B1 region has been synthesised; the new B1 region will displace the B1 region of the stem-loop intermediate). The new B1 region may then form a new stem-loop structure with the new B1c region from which it is separated by the adjacent B2 region. The B2 region may then form a single stranded loop.

Nucleotides may then be added to the 3' end of the F1 region of the stem-loop intermediate. The extension of the F1 region of the stem-loop intermediate will displace the regions newly synthesized by the extension from the F2 region of FIP. This extension may then displace the strand extended from FIP. Once displaced, the stand extended from FIP may form a dumbbell shaped intermediate.

This is because the strand extended from FIP may comprise, in the 5' to 3' direction a F1c region, a F2 region, a LFc region, a F1 region, a B1c region, a LB region, a B2c region and a B1 region. In addition, the addition of nucleotides to the 3' end of B1 may form another stem-loop intermediate.

Both the newly synthesised dumbbell shaped intermediate (i.e. the stand extended from FIP) and the new stem-loop intermediate may serve as templates for a BIP primed strand displacement reaction in a subsequent LAMP cycle.

Similarly, the LAMP cycling may be initiated by BIP and optionally LF hybridising to the loop of the stem-loop intermediate comprising, in the 5' to 3' direction a F1c region, a F2 region, a LFc region, a F1 region, a B1c region, a LB region, a B2c region and a B1 region. In this case, both the newly synthesised dumbbell shaped intermediate (i.e. the stand extended from BIP) and the new stem-loop intermediate may serve as templates for a FIP primed strand displacement reaction in a subsequent LAMP cycle.

The reaction mixture may further comprise a forward outer primer (F3, also known as FOP) and/or a backward outer primer (B3, also known as BOP).

F3 comprises a sequence that is complementary to an F3c region of the nucleic acid. F3 is configured to anneal under stringent conditions to an F3c region of the nucleic acid. The F3c region of the nucleic acid is displaced from the F2c region of the nucleic acid in the 3' direction.

B3 comprises a sequence that is complementary to a B3c region of the nucleic acid. B3 is configured to anneal under stringent conditions to a B3c region of the nucleic acid. The B3c region of the nucleic acid is displaced from the B2c region of the nucleic acid in the 3' direction.

The distance between F2c and F3c, from 3' to 3', may be around 20 to 40 base pairs. The distance between B2c and B3c, from 3' to 3', may be around 20 to 40 base pairs.

The present inventors have determined that detectable amplification of the nucleic acid can occur in the absence of F3 and/or B3. Without being bound by theory this is thought to be possible due to stochastic dissociation of a 5' portion of FIP, such as the F2 region of FIP, after it has been extended by the nucleic acid polymerase. Stochastic dissociation of a 5' portion of extended FIP may allow for annealing of a non-extended FIP. The non-extended FIP may then be extended, displacing the previously extended FIP. Likewise, without being bound by theory, a 5' portion of BIP, such as the B2 region of BIP may also undergo stochastic dissociation after it has been extended by the nucleic acid polymerase. Stochastic dissociation of a 5' portion of extended BIP may allow for annealing of a non-extended BIP. The non-extended BIP may then be extended, displacing the previously extended BIP. However, the inclusion of F3 and/or B3 enhances the efficiency of amplification. This is because F3 does not need to compete with FIP for binding to the nucleic acid. Likewise, B3 does not need to compete with BIP for binding to the nucleic acid.

The reaction mixture may further comprise a forward loop primer (LF) and/or a backward loop primer (LB).

LF comprises a sequence that is corresponds to an LF region of the nucleic acid. The LF region of the nucleic acid is displaced from the F2c region in the 5' direction and is displaced from the F1c region in the 3' direction. Thus, the LF region lies between the F1c and the F2c regions of the nucleic acid. LF is complementary to an LFc region, which is formed by extension of FIP that has annealed to the F2 region of the nucleic acid. LF therefore anneals to the LFc region of the extended FIP under stringent conditions. LF therefore primes extension of extended FIP from an additional site.

LB comprises a sequence that is corresponds to a LB region of the nucleic acid. The LB region of the nucleic acid is displaced from the B2c region in the 5' direction and is displaced from the B1c region in the 3' direction. Thus, the LB region lies between the B1c and the B2c regions of the nucleic acid. LB is complementary to an LBc region, which is formed by extension of BIP that has annealed to the B2 region of the nucleic acid. LB therefore anneals to the LBc region of the extended BIP under stringent conditions. LB therefore primes extension of extended BIP from an additional site.

Detectable amplification may occur in the absence of LF and LB. However, the inclusion of LF and LB enhances the efficiency of amplification. This may be because the loop primers hybridise to loop region of the stem-loop apart from the region which has been hybridised by other LAMP primers.

The primers FIP, BIP, F3, B3, LF and/or BF may be stored as a primer mixture and may therefore be added to the reaction mixture together.

The sample may be any suitable sample comprising a nucleic acid. For example, the sample may be an environmental sample or a clinical sample. The sample may also be a sample of synthetic DNA (such as gBlocks) or a sample of a plasmid. The plasmid may include a gene or gene fragment of interest.

The environmental sample may be a sample from air, water, animal matter, plant matter or a surface. An environmental sample from water may be salt water, brackish water or fresh water. For example, an environmental sample from salt water may be from an ocean, sea or salt marsh. An environmental sample from brackish water may be from an estuary. An environmental sample from fresh water may be from a natural source such as a puddle, pond, stream, river, lake. An environmental sample from fresh water may also be from a man-made source such as a water supply system, a storage tank, a canal or a reservoir. An environmental sample from animal matter may, for example, be from a dead animal or a biopsy of a live animal. An environmental sample from plant matter may, for example, be from a foodstock, a plant bulb or a plant seed. An environmental sample from a surface may be from an indoor or an outdoor surface. For example, the outdoor surface be soil or compost. The indoor surface may, for example, be from a hospital, such as an operating theatre or surgical equipment, or from a dwelling, such as a food preparation area, food preparation equipment or utensils. The environmental sample may contain or be suspected of containing a pathogen. Accordingly, the nucleic acid may be a nucleic acid from the pathogen.

The clinical sample may be a sample from a patient. The nucleic acid may be a nucleic acid from the patient. The clinical sample may be a sample from a bodily fluid. The clinical sample may be from blood, serum, lymph, urine, faeces, semen, sweat, tears, amniotic fluid, wound exudate or any other bodily fluid or secretion in a state of heath or disease. The clinical sample may be a sample of cells or a cellular sample. The clinical sample may comprise cells. The clinical sample may be a tissue sample. The clinical sample may be a biopsy.

The clinical sample may be from a tumour. The clinical sample may comprise cancer cells. Accordingly, the nucleic acid may be a nucleic acid from a cancer cell.

The sample may be obtained by any suitable method. Accordingly, the method of the invention may comprise a step of obtaining the sample. For example, the environmental air sample may be obtained by impingement in liquids, impaction on solid surfaces, sedimentation, filtration, centrifugation, electrostatic precipitation, or thermal precipitation. The water sample may be obtained by containment, by using pour plates, spread plates or membrane filtration. The surface sample may be obtained by a sample/rinse method, by direct immersion, by containment, or by replicate organism direct agar contact (RODAC).

The sample from a patient may contain or be suspected of containing a pathogen. Accordingly, the nucleic acid may be a nucleic acid from the pathogen. Alternatively, the nucleic acid may be a nucleic acid from the host.

The method of the invention may be an *in vitro* method or an *ex vivo* method.

The pathogen may be any entity comprising a nucleic acid comprising a tandem repeat. The pathogen may be a eurkaryote, a prokaryote or a virus. The pathogen may be an animal, a plant, a fungus, a protozoan, a chromist, a bacterium or an archaeum.

The pathogen may be from the pylum *Ascomycota.* The pathogen may be from the class *Eurotiomycetes.* The pathogen may be from the order *Eurotiales.* The pathogen may be from the family *Trichocomaceae.* The pathogen may be from the genus *Aspergillus.* The pathogen may be *Aspergillus fumigatus.*

The pathogen may be from the pylum *Actinobacteria.* The pathogen may be from the order *Actinomycetales.* The pathogen may be from the suborder *Corynebacterineae.* The pathogen may be from the family *Mycobacteriaceae.* The pathogen may be from the genus *Mycobacterium.*

The nucleic acid may be isolated, extracted and/or purified from the sample prior to use in the method of the invention. The isolation, extraction and/or purification may be performed by any suitable technique. For example, the nucleic acid isolation, extraction and/or purification may be performed using a nucleic acid isolation kit, a nucleic acid extraction kit or a nucleic acid purification kit, respectively.

The method of the invention may further comprise an initial step of isolating, extracting and/or purifying the nucleic acid from the sample. The method may therefore further comprise isolating the nucleic acid from the sample. The method may further comprise extracting the nucleic acid from the sample. The method may further comprise purifying the nucleic acid from the sample. Alternatively, the method may comprise direct amplification from the sample without an initial step of isolating, extracting and/or purifying the nucleic acid from the sample. Accordingly, the method may comprise lysing cells in the sample or amplifying free circulating DNA.

Following isolation, extraction and/or purification, the nucleic acid may be used immediately or may be stored under suitable conditions prior to use. Accordingly, the method of the invention may further comprise a step of storing the nucleic acid after the extracting step and before the amplifying step.

The step of obtaining the sample and/or the step of isolating, extracting and/or purifying the nucleic acid from the sample may occur in a different location to the subsequent steps of the method. Accordingly, the method may further comprise a step of transporting the sample and/or transporting the nucleic acid.

It is possible for the method to be performed at the point-of-care. As used herein "at the point-of-care" means in the same or a nearby location to the place where the sample originates. In other words, it may not be necessary to transport the sample, or the nucleic acid, and/or to perform any of the method steps in a location remote from the location at which the sample was obtained. When the sample is a clinical sample, the point-of-care may be the location of the patient from whom the clinical sample was obtained, or a location nearby. When the sample is an environmental sample, the point-of-care may be the location of the air, water, animal matter, plant matter or a surface from whom the environmental sample was obtained, or a location nearby. The method of the invention may be suitable for use at the point-of-care. Accordingly, the method may be described as a method for detecting a tandem repeat in a nucleic acid sequence at the point-of-care. One, more or all of the method steps may be described as being performed at the point-of-care. The amplifying step may be performed at the point-of-care. The detecting step may be performed at the point-of-care. The step of obtaining the sample and/or the step of isolating, extracting and/or purifying the nucleic acid from the sample may be performed at the point-of-care.

Accordingly, the method may be performed extemporaneously. As used herein "extemporaneously" means as soon as the sample is obtained, without delay after the sample is obtained, without transporting the sample, in the same or nearby location to the place where the sample originates and/or at the point-of-care. The method may be performed extemporaneously on the sample. The method may be performed extemporaneously by the same individual who took the sample. For example, the method may be performed extemporaneously by the medical practitioner who took the sample.

A tandem repeat comprises two repeat units. The two repeat units are adjacent to one another in the sequence of the nucleic acid. The tandem repeat may be a direct tandem repeat. Accordingly, the repeat units may appear in the same orientation (i.e. the sequences of each repeat unit correspond in the 5' to 3' direction) rather than in the opposite orientation (i.e. where the sequences correspond when one is read in the 5' to 3' direction and the other is read in the 3' to 5' direction).

The tandem repeat may be an "inverted" tandem repeat. An inverted tandem repeat comprises copies of a DNA sequence that lie adjacent to one another in the opposite direction to one another. Accordingly, the repeat units may appear in the opposite orientation (i.e. the sequences of each repeat unit correspond when one is read in the 5' to 3' direction and the other is read in the 3' to 5' direction) rather than in the same orientation (i.e. the sequences of each repeat unit correspond in the 5' to 3' direction).

The tandem repeat may comprise more than two repeat units. The tandem repeat may comprise two to 50 repeat units. The tandem repeat may comprise two to 30 repeat units. The tandem repeat may comprise two to 20 repeat units. The tandem repeat may comprise two to 10 repeat units. The tandem repeat may comprise three, four, five, six, seven, eight, nine or ten repeat units. The tandem repeat may comprise two or three repeat units.

The tandem repeat may be a microsatellite, a minisatellite, a variable number tandem repeat (VNTR), a MIRU-VNTR (mycobacterial interspersed repetitive unit-variable number tandem repeat, an Alu element or a short interspersed nuclear element (SINE).

A repeat unit may alternatively be termed a repeated nucleic acid sequence motif.

A repeat unit may be any nucleotide sequence from about 2 base pairs to about 200 base pairs in length.

The repeat unit may be from about 2 to 100 base pairs in length. The repeat unit may be from about 2 to 80 base pairs in length. The repeat unit may be from about 2 to 60 base pairs in length. The repeat unit may be from about 2 to 40 base pairs in length. The repeat unit may be from about 2 to 20 base pairs in length. The repeat unit may be from about 2 to 10 base pairs in length.

The repeat unit may be from about 2 to 6 base pairs in length. A repeat unit that is from about 2 to 6 base pairs in length may be termed a microsatellite.

The repeat unit may be from about 5 to 200 base pairs in length. The repeat unit may be from about 5 to 100 base pairs in length. The repeat unit may be from about 5 to 80 base pairs in length. The repeat unit may be from about 5 to 60 base pairs in length. The repeat unit may be from about 5 to 40 base pairs in length. The repeat unit may be from about 5 to 20 base pairs in length. The repeat unit may be from about 5 to 10 base pairs in length.

The repeat unit may be from about 7 to 200 base pairs in length. The repeat unit may be from about 7 to 100 base pairs in length. The repeat unit may be from about 7 to 80 base pairs in length. The repeat unit may be from about 7 to 60 base pairs in length. The repeat unit may be from about 7 to 40 base pairs in length. The repeat unit may be from about 7 to 20 base pairs in length. The repeat unit may be from about 7 to 10 base pairs in length.

The repeat unit may be from about 10 to 200 base pairs in length. The repeat unit may be from about 10 to 100 base pairs in length. The repeat unit may be from about 10 to 80 base pairs in length. The repeat unit may be from about 10 to 60 base pairs in length. The repeat unit may be from about 10 to 40 base pairs in length. The repeat unit may be from about 10 to 20 base pairs in length.

The repeat unit may be from about 13 to 200 base pairs in length. The repeat unit may be from about 13 to 100 base pairs in length. The repeat unit may be from about 13 to 80 base pairs in length. The repeat unit may be from about 13 to 60 base pairs in length. The repeat unit may be from about 13 to 40 base pairs in length. The repeat unit may be from about 13 to 20 base pairs in length.

The repeat unit may be from about 20 to 200 base pairs in length. The repeat unit may be from about 20 to 100 base pairs in length. The repeat unit may be from about 20 to 80 base pairs in length. The repeat unit may be from about 20 to 60 base pairs in length. The repeat unit may be from about 20 to 50 base pairs in length. The repeat unit may be from about 30 to 50 base pairs in length. The repeat unit may be from about 34 to 46 base pairs in length. The repeat unit may be from about 29 to 39 or from about 41 to 51 base pairs in length. The repeat unit may be from about 31 to 37 or from about 43 to 49 base pairs in length. The repeat unit may be about 34 or about 46 base pairs in length.

The repeat unit may be from about 10 to 60 base pairs in length. A repeat unit that is from about 10 to 60 base pairs in length may be termed a minisatellite. The repeat unit may be about 30 base pairs in length. A repeat unit that is about 30 base pairs in length may also be termed a minisatellite.

When the repeat unit is about 30 to 50 base pairs in length, the tandem repeat may comprise two or three repeat units. When the repeat unit is about 30 base pairs in length, the tandem repeat may comprise two or three repeat units.

The repeat unit may be from about 10 to 100 base pairs in length. A repeat unit that is from about 10 to 100 base pairs in length may be termed a VNTR.

The repeat unit may be from about 100 to 200 base pairs in length. The repeat unit may be from about 120 to about 200 base pairs in length. The repeat unit may be from about 140 to about 200 base pairs in length. The repeat unit may be from about 160 to 200 base pairs in length. The repeat unit may be from about 180 to 200 base pairs in length.

The repeat unit may be from about 100 to 300 base pairs in length. A repeat unit that is from about 100 to 300 base pairs in length may be termed a SINE. SINEs may extent from about 100 to 700 base pairs. The method of the invention may be used to detect SINEs from about 100 to 300 base pairs in length.

The two or more repeat units may have the same nucleic acid sequence. The nucleic acid sequence of the repeat units may be 100% identical. However, the two or more repeat units may not have the same nucleic acid sequence. The nucleic acid sequence of the repeat units may not be 100% identical. The repeat units may comprise one or more differences in nucleic acid sequence such as one or more insertions, one or more deletions and/or one or more point mutations. The nucleic acid sequence of the repeat units may be 50 to 100% identical. The nucleic acid sequence of the repeat units may be 60 to 100% identical. The nucleic acid sequence of the repeat units may be 70 to 100% identical. The nucleic acid sequence of the repeat units may be 80 to 100% identical. The nucleic acid sequence of the repeat units may be 90 to 100% identical. The nucleic acid sequence of the repeat units may be 95 to 100% identical.

The target region comprises one or more nucleotides from each of the repeat units. The target region may comprise two or more, three or more, four or more, five or more, six or more, seven or more, eight or more, nine or more, or ten or more nucleotides from each of the repeat units. The number of nucleotides of the target region from the each of the repeat units may be the same. Accordingly, the two repeat units may contribute equally to the target region. Alternatively, the number of nucleotides of the target region from the each of the repeat units may be the different. Accordingly, the two repeat units may contribute unequally to the target region.

The two repeat units contributing to the target region are adjacent, or consecutive. The two repeat units may be referred to as a first repeat unit and a second repeat unit. The terms "first" and "second" may be used interchangeably with respect to the repeat units. Alternatively, the "first" repeat unit may be displaced in the 5' direction from the second repeat unit. Stated another way, the "second" repeat unit may be displaced from the first repeat unit in the 3' direction.

The target region overlaps at least a portion of the two repeat units. The target region may also be described as a "joining region", accordingly.

The target region may comprise one or more nucleotides from a first repeat unit and one or more nucleotides from a second repeat unit. The target region may comprise one to 25 nucleotides from the first repeat unit and one to 25 nucleotides from the second repeat unit. The target region may comprise one to 25 nucleotides from the first repeat unit and one to 10 nucleotides from the second repeat unit. The target region may comprise one to 25 nucleotides from the first repeat unit and one to five nucleotides from the second repeat unit. The target region may comprise one to 25 nucleotides from the first repeat unit and one to four nucleotides from the second repeat unit. The target region may comprise one to 25 nucleotides from the first repeat unit and one to three nucleotides from the second repeat unit. The target region may comprise one to 25 nucleotides from the first repeat unit and one or two nucleotides from the second repeat unit.

The target region may comprise two or more nucleotides from the first repeat unit and one or more nucleotides from the second repeat unit. The target region may comprise three or more, four or more, five or more, six or more, seven or more, eight or more, nine of more, ten or more, 11 or more, 12 or more, 13 or more, 14 or more, 15 or more, 16 or more, 17 or more, 18 or more, 19 or more, 20 or more, 21 or more, 22 or more, 23 or more, 24 or more, or 25 or more nucleotides from the first repeat unit and one or more nucleotides from the second repeat unit.

The target region may comprise two or more nucleotides from the first repeat unit and two or more nucleotides from the second repeat unit. The target region may comprise three or more, four or more, five or more, six or more, seven or more, eight or more, nine of more, ten or more, 11 or more, 12 or more, 13 or more, 14 or more, 15 or more, 16 or more, 17 or more, 18 or more, 19 or more, 20 or more, 21 or more, 22 or more, 23 or more, 24 or more, or 25 or more nucleotides from the first repeat unit and two or more nucleotides from the second repeat unit.

The target region may comprise two or more nucleotides from the first repeat unit and three or more nucleotides from the second repeat unit. The target region may comprise three or more, four or more, five or more, six or more, seven or more, eight or more, nine of more, ten or more, 11 or more, 12 or more, 13 or more, 14 or more, 15 or more, 16 or more, 17 or more, 18 or more, 19 or more, 20 or more, 21 or more, 22 or more, 23 or more, 24 or more, or 25 or more nucleotides from the first repeat unit and three or more nucleotides from the second repeat unit.

The target region may comprise two or more nucleotides from the first repeat unit and four or more nucleotides from the second repeat unit. The target region may comprise three or more, four or more, five or more, six or more, seven or more, eight or more, nine of more, ten or more, 11 or more, 12 or more, 13 or more, 14 or more, 15 or more, 16 or more, 17 or more, 18 or more, 19 or more, 20 or more, 21 or more, 22 or more, 23 or more, 24 or more, or 25 or more nucleotides from the first repeat unit and four or more nucleotides from the second repeat unit.

The target region may comprise two or more nucleotides from the first repeat unit and five or more nucleotides from the second repeat unit. The target region may comprise three or more, four or more, five or more, six or more, seven or more, eight or more, nine of more, ten or more, 11 or more, 12 or more, 13 or more, 14 or more, 15 or more, 16 or more, 17 or more, 18 or more, 19 or more, 20 or more, 21 or more, 22 or more, 23 or more, 24 or more, or 25 or more nucleotides from the first repeat unit and five or more nucleotides from the second repeat unit.

The tandem repeat may be less than or equal to 300 base pairs long. The tandem repeat may be from 2 to 300 base pairs long. The tandem repeat may be from 20 to 70 base pairs long. The tandem repeat may be from 20 to 50 base pairs long. The tandem repeat may be from 30 to 70 base pairs long. The tandem repeat may be from 30 to 50 base pairs long. The tandem repeat may be from 50 to 70 base pairs long.

The target region may comprise a number of nucleotides from the first repeat unit equal to or greater than the number of nucleotides from the second repeat unit. The target region may comprise a number of nucleotides from the first repeat unit greater than the number of nucleotides from the second repeat unit.

All of the nucleotides contributing to the target region may be nucleotides from the first and second repeat units. Accordingly, all of the nucleotides contributing to the target region may be nucleotides from the tandem repeat.

The total number of nucleotides comprising the target region may be two to 100. If the target region is defined as the distance between F1c and F2c, the total number of nucleotides comprising the target region may be two 0 to 100. The total number of nucleotides comprising the target region may be two to 50. The total number of nucleotides comprising the target region may be two to 25. The total number of nucleotides comprising the target region may be two, tree, four, five, six, seven, eight, nine, ten, 11, 12, 13, 14, 15, 16, 17, 18, 19, 20, 21, 22, 23, 24 or 25.

The FIP may anneal to a target region comprising 10 or more nucleotides from a first repeat unit; one or more nucleotides from a second repeat unit; and 18 or more nucleotides in total. The FIP may anneal to a target region comprising 10 or more nucleotides from a first repeat unit; one or more nucleotides from a second repeat unit; and 19 or more nucleotides in total. The FIP may anneal to a target region comprising 10 or more nucleotides from a first repeat unit; one or more nucleotides from a second repeat unit; and 20 or more nucleotides in total. The FIP may anneal to a target region comprising 10 or more nucleotides from a first repeat unit; one or more nucleotides from a second repeat unit; and 21 or more nucleotides in total. The FIP may anneal to a target region comprising 10 or more nucleotides from a first repeat unit; one or more nucleotides from a second repeat unit; and 22 or more nucleotides in total. The FIP may anneal to a target region comprising 10 or more nucleotides from a first repeat unit; one or more nucleotides from a second repeat unit; and 23 or more nucleotides in total. The FIP may anneal to a target region comprising 10 or more nucleotides from a first repeat unit; one or more nucleotides from a second repeat unit; and 24 or more nucleotides in total.

The FIP may anneal to a target region comprising 10 or more nucleotides from a first repeat unit; one or more nucleotides from a second repeat unit; and 15 to 25 nucleotides in total. The FIP may anneal to a target region comprising 10 or more nucleotides from a first repeat unit; one or more nucleotides from a second repeat unit; and 16 to 24 nucleotides in total. The FIP may anneal to a target region comprising 10 or more nucleotides from a first repeat unit; one or more nucleotides from a second repeat unit; and 17 to 22 nucleotides in total. The FIP may anneal to a target region comprising 10 or more nucleotides from a first repeat unit; one or more nucleotides from a second repeat unit; and 18 to 40 nucleotides in total.

The FIP may anneal to a target region comprising 10 or more nucleotides from a first repeat unit; one to 10 nucleotides from a second repeat unit; and 18 to 40 or more nucleotides in total.

The FIP may anneal to a target region comprising 10 or more nucleotides from a first repeat unit; one to 10 nucleotides from a second repeat unit; and 19 or more nucleotides in total.

The F2 region of FIP may anneal under stringent conditions to any target region as defined herein. Accordingly, the F2 region of FIP may comprise 10 to 50 nucleotides. The total number of nucleotides comprising the F2 region of FIP may be 18 to 30. The total number of nucleotides comprising the F2 region of FIP may be 18, 19, 20, 21, 22, 23, 24, 25, 26, 27, 28, 29 or 30.

One embodiment of the invention provides a method for detecting a tandem repeat in a nucleic acid sequence, wherein the tandem repeat comprises two or more repeat units each of which comprises at least two base pairs, wherein the method comprises

(a) amplifying under isothermal conditions and stringent conditions a nucleic acid sequence from a sample, in a reaction mixture comprising

(i) the nucleic acid sequence,
(ii) a nucleic acid polymerase,
(iii) a nucleoside triphosphate mixture,
(iv) a forward inner primer (FIP),
(v) a backward inner primer (BIP),
(vi) a forward outer primer (F3),
(vii) a backward outer primer (B3), and
(viii) a forward loop primer (LF) and/or a backward loop primer (LB)

wherein the FIP or BIP anneals to a target region comprising 10 or more nucleotides from a first repeat unit; one to 10 nucleotides from a second repeat unit; and 18 to 30 nucleotides in total, wherein the two repeat units are adjacent, and
(b) detecting an amplified nucleic acid sequence, wherein the detection of an amplified nucleic acid sequence indicates the presence the tandem repeat.

Another embodiment of the invention provides a method for detecting a tandem repeat in a nucleic acid sequence, wherein the tandem repeat comprises two or more repeat units each of which comprises 20 to 200 base pairs, wherein the method comprises

(a) amplifying under isothermal conditions and stringent conditions a nucleic acid sequence from a sample, in a reaction mixture comprising

(i) the nucleic acid sequence,
(ii) a nucleic acid polymerase,
(iii) a nucleoside triphosphate mixture,
(iv) a forward inner primer (FIP),
(v) a backward inner primer (BIP),
(vi) a forward outer primer (F3),
(vii) a backward outer primer (B3), and
(viii) a forward loop primer (LF) and/or a backward loop primer (LB)

wherein the FIP or BIP anneals to a target region comprising 10 or more nucleotides from a first repeat unit; one to 10 nucleotides from a second repeat unit; and 18 to 30 nucleotides in total, wherein the two repeat units are adjacent, and
(b) detecting an amplified nucleic acid sequence, wherein the detection of an amplified nucleic acid sequence indicates the presence the tandem repeat.

The tandem repeat may be associated with, contribute to and/or define drug resistance in the pathogen. For example, when the pathogen is Aspergillus fumigatus, the tandem repeat may be associated with, contribute to and/or define azole resistance. When the pathogen is Aspergillus fumigatus, the tandem repeat may be in the *cyp51A* gene. For example, the tandem repeat may be a 34 base pair tandem repeat in the *cyp51A* gene of *A. fumigatus,* named TR34; a 46 base pair tandem repeat in the *cyp51A* gene of *A. fumigatus,* named TR46; or a 53 base pair tandem repeat in the *cyp51A* gene of *A. fumigatus,* named TR53.

As used herein, the term "drug" refers to a therapeutic or prophylactic agent. The drug may therefore be an active ingredient in a pharmaceutical composition or in a pharmaceutical formulation. The drug may be a pharmaceutical composition or in a pharmaceutical formulation. The drug may be a combination therapy.

As used herein the term "drug resistance" refers to the ability of an organism, such as a pathogen, to survive exposure to a drug administered to a patient to treat an infection or condition or disease caused by the pathogen. For example, such drugs may be characterised as pesticides, fungicides, viricides, antibiotics, antiparasitics or antimicrobial drugs. An organism exhibiting "drug resistance" may be described as "drug resistant". Drug resistance may be measured by comparison with a control organism which has undergone the same treatment with the same drug. When compared with a control organism the organism suspected of being drug resistant may be described as the "test" organism. A sample of the test organism and a sample of the control organism may be compared. Any suitable measure may be used. For example, the drug resistant organism may survive longer, grow at a faster rate, divide or proliferate at a faster rate and/or die at a slower rate than the control organism.

Drug resistance may be contributed to and/or defined by DNA sequence and/or gene expression. For example, when a DNA sequence is known to contribute to and/or define drug resistance the presence or absence of the DNA sequence may be determined by any suitable technique. Likewise, when gene expression is known to contribute to and/or define drug resistance, the gene expression may be determined by any suitable technique. Drug resistance may be contributed to and/or defined by a tandem repeat. When drug resistance is contributed to and/or defined by a tandem repeat, a method of detecting drug resistance may comprise detecting the tandem repeat in accordance with the first aspect of the invention.

An infection may also be described as drug resistant if it has the ability to survive treatment with a drug. A drug resistant infection may comprise a drug resistant organism, such as a drug resistant pathogen. The test sample may be from the infection by any suitable means, such as a swab or a biopsy or any means described herein in connection with a clinical sample.

When the tandem repeat is TR34, the primer sequences may comprise the following primer sequences (written in the 5' to 3' direction):

- FIP may comprise TAATTAGGCAACTTTCATTCCGGATGTGTGCTGAGCCGAATAAAT (SEQ ID NO: 1)
- BIP may comprise ACTAAGGTGTAGTTCCAGCATACCGTAAGTAGATCTACCTACCGTAGT (SEQ ID NO: 2)
- F3 may comprise CACCACTTCAGAGTTGTCT (SEQ ID NO: 3)
- B3 may comprise GTATTTTATATTCACCTACCTACCA (SEQ ID NO: 4)
- LF may comprise CGGACCGCGTGATTCAT (SEQ ID NO: 5).

When the tandem repeat is TR46, the primer sequences may comprise the following primer sequences (written in the 5' to 3' direction):

- FIP may comprise

  TAATTAGGCAACTTTCATTCCGGATGTGTGCTGAGAAAAAGGAAAGTTGTCTAG (SEQ ID NO: 6)

- BIP may comprise ACTAAGGTGTAGTTCCAGCATACCGTAAGTAGATCTACCTACCGTAGT (SEQ ID NO: 7)
- F3 may comprise CACCACTTCAGAGTTGTCT (SEQ ID NO: 8)
- B3 may comprise GTATTTTATATTCACCTACCTACCA (SEQ ID NO: 9)
- LB may comprise ACCATACACCCTAACTCATACTACGG (SEQ ID NO: 10).

Alternatively, when the nucleic acid is a nucleic acid from the host, the tandem repeat may contribute to or define a characteristic of the host. The characteristic of the host may be susceptibility to infection.

Accordingly, the method may alternatively be defined as a method of screening for susceptibility to infection by detecting a tandem repeat in a nucleic acid sequence; a method of diagnosing susceptibility to infection by detecting a tandem repeat in a nucleic acid sequence; and/or a method for prophylactically treating infection comprising detecting a tandem repeat in a nucleic acid sequence and prophylactically treating an infection when the tandem repeat is present.

When the nucleic acid is from a clinical sample, the tandem repeat may contribute to or define disease, disorder or trait risk. The disease disorder or trait may be any disease disorder or trait to which a tandem repeat is known to contribute or define risk.

The tandem repeat may be a tandem repeat in the human genome.

The tandem repeat may contribute to or define risk of a tandem repeat polymorphism associated disorder. For example, the tandem repeat may be in the 5-HTT gene. The tandem repeat in the 5-HTT gene may contribute to or define risk of depression, anxiety disorders and/or Bipolar disorder.

The tandem repeat may contribute to or define risk of a neurological event, such as stroke. For example, the tandem repeat may be in the GPIba gene. The tandem repeat in the GPIba gene may contribute to or define risk of recurrent events in stroke patients treated with aspirin. A tandem repeat in the GPIba gene can be an independent predictor of recurrent events in stroke patients treated with aspirin

When the tandem repeat is a tandem repeat in the human genome, detection of the tandem repeat may be used in a method of DNA fingerprinting.

The tandem repeat may be a MIRU-VNTR (mycobacterial interspersed repetitive unit-variable number of tandem repeat) associated with drug resistance of Mycobacterium tuberculosis.

Another embodiment of the invention provides a method for detecting a tandem repeat in a nucleic acid sequence, wherein the tandem repeat is a TR34 mutation of *A. fumigatus,* wherein the method comprises

(a) amplifying under isothermal conditions and stringent conditions a nucleic acid sequence from a sample of *A. fumigatus,* in a reaction mixture comprising

(i) the nucleic acid sequence,
(ii) a nucleic acid polymerase,
(iii) a nucleoside triphosphate mixture,
(iv) a forward inner primer (FIP),
(v) a backward inner primer (BIP),
(vi) a forward outer primer (F3),
(vii) a backward outer primer (B3), and
(viii) a forward loop primer (LF) and/or a backward loop primer (LB)

wherein the FIP or BIP anneals to a target region comprising 20 or more nucleotides from a first repeat unit; one to 10 nucleotides from a second repeat unit; and 18 to 40 nucleotides in total, wherein the two repeat units are adjacent, and
(b) detecting an amplified nucleic acid sequence, wherein the detection of an amplified nucleic acid sequence indicates the presence the tandem repeat.

Another embodiment of the invention provides a method for detecting a tandem repeat in a nucleic acid sequence, wherein the tandem repeat is a TR46 mutation of *A. fumigatus,* wherein the method comprises

(a) amplifying under isothermal conditions and stringent conditions a nucleic acid sequence from a sample of *A. fumigatus,* in a reaction mixture comprising

(i) the nucleic acid sequence,
(ii) a nucleic acid polymerase,
(iii) a nucleoside triphosphate mixture,
(iv) a forward inner primer (FIP),
(v) a backward inner primer (BIP),
(vi) a forward outer primer (F3),
(vii) a backward outer primer (B3), and
(viii) a forward loop primer (LF) and/or a backward loop primer (LB)

wherein the FIP or BIP anneals to a target region comprising 15 or more nucleotides from a first repeat unit; one to 10 nucleotides from a second repeat unit; and 18 to 40 nucleotides in total, wherein the two repeat units are adjacent, and
(b) detecting an amplified nucleic acid sequence, wherein the detection of an amplified nucleic acid sequence indicates the presence the tandem repeat.

On-site diagnosis of the TR34 resistance allele is important, since it will allow the clinician to make more rapid decisions as to how to manage the patients' infections. The current diagnosis of invasive aspergillosis includes direct examination of respiratory specimens, culture of the organism, histopathological examination, galactomannan antigen detection, beta-D-glucan assay and molecular-based detection (such as PCR) techniques. However, the sensitivity of current diagnostic methods for IA is generally low and may even return a negative result for some individuals at high risk. Apart from their low sensitivity and specificity, other disadvantages of these methods are that they are time-consuming and rely on laboratory-based instruments.

Compared to the current methods, the method of the invention, based on LAMP chemistry, has the advantage of a short amplification time with high sensitivity and specificity. Moreover, PCR and other molecular methods can only be conducted in well-equipped laboratories and thus are impracticable in resource limited settings, whereas LAMP does not involve thermal cycling steps and is able to amplify target DNAs in a short period of time, to detectable levels. Another advantage of the LAMP assay is that the results can qualitatively be detected by the naked eye, and thus this makes it a suitable technique for a resource-limited setting. These advantages of the LAMP assay enable a time-saving and cost-effective method, in comparison to conventional laboratory-based and fluorescence-based methods.

A rapid and highly specific LAMP reaction for TR34 detection has been established. This TR34-LAMP assay shows exceptionally high sensitivity (10 copies/reaction) within only 30 minutes reaction. This may be achieved by applying four primers which are targeting six regions of a DNA template, and one loop primer to accelerate the reaction. Full-complementarity between the DNA template and primer sequence is generally considered essential for a specific amplification; therefore, introducing primer-template mismatches at the 3'end of a primer has the potential to increase the amplification discrimination significantly between similar targets. In this study, the challenge in designing the primers was to place them in a long direct tandem repeat section that is flanked by highly conserved regions. In order to increase the amplification discrimination, primer F2 was constructed based on the sequence of the junction between the tandem repeat mutation and the conserved region, and a primer-template G-A mismatch was introduced at 3'end of F2. The results showed that by replacing this single mismatch at the 3'end of F2, only the TR34 strain was amplified, instead of showing a time to positive (TTP) delay between TR34 and TR46 amplifications. The TR-LAMP method disclosed herein can be applied to the detection of other tandem repeat mutations, such as TR46 and TR53 *cyp51A* mutations. A further potential use of TR-LAMP is for on-site forensic detection, which usually detecting hypervariable tandem repeats, minisatellites (about 30 bp per repeat unit) variation among individuals. The method disclosed herein combined with lab-on-chip platform also enables a "fingerprint" detection device as a digital forensics tool. The combination of the method of the invention with a lab-on-chip platform, allows all the analytical steps to be conducted in a rapid, effective and automated manner.

The one-step TR34-LAMP assay disclosed herein is useful for the rapid diagnosis of the TR34 mutation in the *cyp51A* gene of *A. fumigatus.* Due to its high reliability, fast identification and ease of use, this method may determine the prevalence of the TR34 mutation in both environmental and clinical settings.

According to a second aspect, the invention provides a method for detecting drug resistance in an organism by detecting a tandem repeat in a nucleic acid sequence, wherein the tandem repeat comprises two or more repeat units each of which comprises at least two base pairs, wherein the method comprises

(a) amplifying under isothermal conditions and stringent conditions a nucleic acid sequence from a sample, in a reaction mixture comprising

(i) the nucleic acid sequence,
(ii) a nucleic acid polymerase,
(iii) a nucleoside triphosphate mixture,
(iv) a forward inner primer (FIP), and
(v) a backward inner primer (BIP),

wherein the FIP or BIP anneals to a target region comprising one or more nucleotides from each of two repeat units, wherein the two repeat units are adjacent, and

(b) detecting an amplified nucleic acid sequence, wherein the detection of an amplified nucleic acid sequence indicates the presence the tandem repeat.

The method for detecting drug resistance in an organism may therefore be defined as a method for detecting drug resistance in an organism, the method comprising detecting a tandem repeat in a nucleic acid sequence according to the method of the first aspect of the invention.

The tandem repeat may define and/or contribute to drug resistance. The tandem repeat may therefore be any tandem repeat known in the art to define and/or contribute to drug resistance. For example, the tandem repeat may be selected from the group consisting of TR34 in the *cyp51A* gene of *A. fumigatus;* TR46 in the *cyp51A* gene of *A. fumigatus;* and TR53 in the *cyp51A* gene of *A. fumigatus.*

According to a third aspect, the invention provides a method for the diagnosis of an infectious disease or a drug resistant infection by detecting a tandem repeat in a nucleic acid sequence, wherein the tandem repeat comprises two or more repeat units each of which comprises at least two base pairs, wherein the method comprises

(a) amplifying under isothermal conditions and stringent conditions a nucleic acid sequence from a sample, in a reaction mixture comprising

(i) the nucleic acid sequence,
(ii) a nucleic acid polymerase,

(iii) a nucleoside triphosphate mixture,
(iv) a forward inner primer (FIP), and
(v) a backward inner primer (BIP),

wherein the FIP or BIP anneals to a target region comprising one or more nucleotides from each of two repeat units, wherein the two repeat units are adjacent, and
(b) detecting an amplified nucleic acid sequence, wherein the detection of an amplified nucleic acid sequence indicates the presence the tandem repeat.

The method for the diagnosis of an infectious disease or a drug resistant infection may therefore be defined as a method for the diagnosis of an infectious disease or a drug resistant infection comprising detecting a tandem repeat in a nucleic acid sequence according to the method of the first aspect of the invention.

The tandem repeat may contribute to and/or define the infectious disease or the drug resistant infection.

The tandem repeat may be a biomarker for the infectious disease or the drug resistant infection. For example, the tandem repeat may be selected from the group consisting of TR34 in the *cyp51A* gene of *A. fumigatus;* TR46 in the *cyp51A* gene of *A. fumigatus;* and TR53 in the *cyp51A* gene of *A. fumigatus,* each of which is a biomarker for drug resistant *A. fumigatus.* Tandem repeats which are biomarkers for infectious diseases and drug resistant infections are known.

A "biomarker" is a naturally occurring molecule, gene, or characteristic by which a particular pathological or physiological process, disease, etc. can be identified. A biomarker may be a measurable indicator by which a particular pathological or physiological process, disease, etc. can be identified. Accordingly, a biomarker may be a measurable indicator of the presence of an infectious disease or drug resistant infection. The biomarker may increase or decrease in concentration in a sample when the infectious disease or drug resistant infection is present. For example, a tandem repeat may be present at a higher concentration when the sample is from a subject with an infectious disease or drug resistant infection than in a control sample. The relevant control sample may be from a different subject. Alternatively, the control sample may a different sample from the same subject, such as a sample from another location or time point. The other location may be, for example, a non-infected region or a different infected region of the same subject. The other time point may for example be an earlier or a later time point when the infectious disease or drug resistant infection was not present and/or not symptomatic.

The method may further comprise diagnosing an infectious disease or a drug resistant infection if the tandem repeat is present.

The method of diagnosis may be an *in vitro* method or an *ex vivo* method.

According to a fourth aspect, a method for the treatment of an infectious disease or drug resistant infection in a subject in need thereof, is described herein (not claimed) the method comprising detecting a tandem repeat in a nucleic acid sequence, wherein the tandem repeat comprises two or more repeat units each of which comprises at least two base pairs, wherein the method comprises

(a) amplifying under isothermal conditions and stringent conditions a nucleic acid sequence from a sample, in a reaction mixture comprising

(i) the nucleic acid,
(ii) a nucleic acid polymerase,
(iii) a nucleoside triphosphate mixture,
(iv) a forward inner primer (FIP), and
(v) a backward inner primer (BIP),

wherein the FIP or BIP anneals to a target region comprising one or more nucleotides from each of two repeat units, wherein the two repeat units are adjacent, and
(b) detecting an amplified nucleic acid sequence, wherein the detection of an amplified nucleic acid sequence indicates the presence the tandem repeat, and
(c) administering a drug to the subject in need thereof.

The method for the treatment of an infectious disease or a drug resistant infection may therefore be defined as a method for the treatment of an infectious disease or a drug resistant infection in a subject in need thereof, the method comprising detecting a tandem repeat in a nucleic acid sequence according the method of the first aspect of the invention, further comprising administering a drug to the subject in need thereof.

The method for treatment of an infectious disease or a drug resistant infection may also be defined as a method for the treatment of an infectious disease or a drug resistant infection in a subject in need thereof, the method comprising diagnosing an infectious disease or a drug resistant infection according the method of the third aspect of the invention, further comprising administering a drug to the subject in need thereof.

Treating the drug resistant infection may comprise administration of a therapeutic agent. The therapeutic agent may be an alternative therapeutic agent, for example a therapeutic agent to which the infection is not resistant. The therapeutic agent may be selected by a medical practitioner on the basis that resistance to the therapeutic agent is not associated with, contributed to and/or defined by the tandem repeat.

For example, when the drug resistant infection is an azole resistant infection, for instance when the tandem repeat is TR34 or TR46, then the therapeutic agent may be a non-azole therapeutic agent. Examples of non-azole therapeutic agents that can be used to treat an Aspergillus infection include lipid amphotericin formulations, caspofungin, and micafungin.

The infectious disease may be caused by one or more pathogenic microorganisms, such as bacteria, viruses, parasites or fungi. Infectious diseases can be spread, directly or indirectly, from one person to another. The infectious disease may be a zoonotic diseases, which is an infectious diseases of animals that can cause disease when transmitted to humans.

The infectious disease may be selected from the group consisting of Acute Flaccid Myelitis (AFM), , Anaplasmosis, Anthrax, Babesiosis, Botulism, Brucellosis, Burkholderia mallei (Glanders), Burkholderia pseudomallei (Melioidosis), Campylobacteriosis (Campylobacter), Carbapenem-resistant Infection (CRE/CRPA), Chancroid, Chikungunya Virus Infection (Chikungunya), Chlamydia, Ciguatera, Clostridium Difficile Infection, Clostridium Perfringens (Epsilon Toxin), Coccidioidomycosis fungal infection (Valley fever), Creutzfeldt-Jacob Disease , transmissible spongiform encephalopathy (CJD), Cryptosporidiosis (Crypto), Cyclosporiasis, Dengue , 1,2,3,4 (Dengue Fever), Diphtheria, E. coli infection (E.Coli), Eastern Equine Encephalitis (EEE), Ebola, Hemorrhagic Fever (Ebola), Ehrlichiosis, Encephalitis , Arboviral or parainfectious, Enterovirus Infection , Non-Polio (Non-Polio Enterovirus), Enterovirus Infection , D68 (EV-D68), Giardiasis (Giardia), Gonococcal Infection (Gonorrhea), Granuloma inguinale, Haemophilus Influenza disease , Type B (Hib or H-flu), Hantavirus Pulmonary Syndrome (HPS), Hemolytic Uremic Syndrome (HUS), Hepatitis A (Hep A), Hepatitis B (Hep B), Hepatitis C (Hep C), Hepatitis D (Hep D), Hepatitis E (Hep E), Herpes, Herpes Zoster , zoster VZV (Shingles), Histoplasmosis infection (Histoplasmosis), Human Immunodeficiency Virus/AIDS (HIV/AIDS), Human Papillomarivus (HPV), Influenza (Flu), Legionellosis (Legionnaires Disease), Leprosy (Hansens Disease), Leptospirosis, Listeriosis (Listeria), Lyme Disease, Lymphogranuloma venereum infection (LVG), Malaria, Measles, Meningitis , Viral (Meningitis, viral), Meningococcal Disease , Bacterial (Meningitis, bacterial), Middle East Respiratory Syndrome Coronavirus (MERS-CoV), Mumps, Norovirus, Paralytic Shellfish Poisoning (Paralytic Shellfish Poisoning, Ciguatera), Pediculosis (Lice, Head and Body Lice), Pelvic Inflammatory Disease (PID), Pertussis (Whooping Cough), Plague; Bubonic, Septicemic, Pneumonic (Plague), Pneumococcal Disease (Pneumonia), Poliomyelitis (Polio), Powassan, Psittacosis, Pthiriasis (Crabs; Pubic Lice Infestation), Pustular Rash diseases (Small pox, monkeypox, cowpox), Q-Fever, Rabies, Ricin Poisoning, Rickettsiosis (Rocky Mountain Spotted Fever), Rubella , Including congenital (German Measles), Salmonellosis gastroenteritis (Salmonella), Scabies Infestation (Scabies), Scombroid, Severe Acute Respiratory Syndrome (SARS), Shigellosis gastroenteritis (Shigella), Smallpox, Staphyloccal Infection , Methicillin-resistant (MRSA), Staphylococcal Food Poisoning , Enterotoxin - B Poisoning (Staph Food Poisoning), Staphylococcal Infection , Vancomycin Intermediate (VISA), Staphylococcal Infection , Vancomycin Resistant (VRSA), Streptococcal Disease , Group A (invasive) (Strep A), Streptococcal Disease , Group B (Strep-B), Streptococcal Toxic-Shock Syndrome , STSS, Toxic Shock (STSS, TSS), Syphilis , primary, secondary, early latent, late latent, congenital, Tetanus Infection , tetani (Lock Jaw), Trichonosis Infection (Trichinosis), Tuberculosis (TB), Tuberculosis (Latent) (LTBI), Tularemia (Rabbit fever), Typhoid Fever , Group D, Typhus, Vaginosis , bacterial (Yeast Infection), Varicella (Chickenpox), Vibrio cholerae (Cholera), Vibriosis (Vibrio), Viral Hemorrhagic Fever (Ebola, Lassa, Marburg), West Nile Virus, Yellow Fever, Yersenia (Yersinia) and Zika Virus Infection (Zika).

The tandem repeat may be a biomarker for the infectious disease. Tandem repeats which are biomarkers for infectious diseases are known.

The invention further provides a method for point-of-care diagnosis of an infectious disease by detecting a tandem repeat in a nucleic acid sequence, wherein the tandem repeat comprises two or more repeat units each of which comprises at least two base pairs, wherein the method comprises

(a) amplifying under isothermal conditions and stringent conditions a nucleic acid sequence from a sample, in a reaction mixture comprising

(i) the nucleic acid sequence,
(ii) a nucleic acid polymerase,
(iii) a nucleoside triphosphate mixture,
(iv) a forward inner primer (FIP), and
(v) a backward inner primer (BIP),

wherein the FIP or BIP anneals to a target region comprising one or more nucleotides from each of two repeat units, wherein the two repeat units are adjacent, and

(b) detecting an amplified nucleic acid sequence, wherein the detection of an amplified nucleic acid sequence indicates the presence the tandem repeat.

The method for point-of-care diagnosis of an infectious disease may optionally further comprise diagnosing an infectious disease if the tandem repeat is present.

The method for point-of-care diagnosis of an infectious disease may therefore be defined as a method for point-of-care diagnosis of an infectious disease by detecting a tandem repeat in a nucleic acid sequence, comprising the method of the first aspect of the invention, further comprising diagnosing an infectious disease if the tandem repeat is present.

According to a fifth aspect, the invention provides a kit comprising

(i) a forward inner primer (FIP), and
(ii) a backward inner primer (BIP),

wherein the FIP or BIP is configured to anneal under stringent conditions to a target region comprising one or more nucleotides from each of two repeat units in a tandem repeat, wherein the two repeat units are adjacent and each of the two repeat units comprises at least two nucleotides.

As used herein, a "kit" is a packaged combination optionally including instructions for use of the combination and/or other reactions and components for such use.

The kit may further comprise a forward outer primer (F3) and/or a backward outer primer (B3).

The kit may further comprise a forward loop primer (LF) and/or a backward loop primer (LB).

For example, the kit may comprise:

(i) a forward inner primer (FIP),
(ii) a backward inner primer (BIP),
(iii) a forward outer primer (F3),
(iv) a backward outer primer (B3), and
(v) a forward loop primer (LF),

wherein the FIP or BIP is configured to anneal under stringent conditions to a target region comprising one or more nucleotides from each of two repeat units in a tandem repeat, wherein the two repeat units are adjacent and each of the two repeat units comprises at least two nucleotides.

The kit may further comprise a nucleic acid polymerase and/or a nucleoside triphosphate mixture.

Any of the kit components defined above may be combined in any combination.

According to a sixth aspect, the invention provides a reaction mixture comprising the components of the kit according to the fifth aspect of the invention in a liquid medium.

The invention also provides a use of the kit according to the fifth aspect of the invention or of the reaction mixture according to the sixth aspect of the invention in the detection of a pathogen.

The present invention will now be described by way of reference to the following Examples and accompanying Drawings which are present for the purposes of illustration only and are not to be construed as being limiting on the invention.

**Example 1** - **TR34-LAMP assay**

**Samples and DNA extraction method**

Synthetic DNA containing the TR34 and TR46 target were synthesised by Integrated DNA Technologies. High-molecular-weight DNA was extracted from *A. fumigatus* conidia using the MasterPure yeast DNA purification kit (Lucigen, Middleton, WI) according to the manufacturer's instructions but with an additional beading beating step. Harvested conidia were disrupted using 1.0-mm-diameter zirconia/silica beads (BioSpec Products, Bartlesville, OK) in a Qiagen TissueLyser II set to 30 rpm/sec for 45 sec (Qiagen, Hilden, Germany). The gDNA was quantified using a Qubit 3.0 fluorometer and the high sensitivity double-stranded DNA assay kit (Life Technologies, Carlsbad, CA). Integrity of the gDNA was confirmed using a TapeStation 2200 system and gDNA ScreenTape (Agilent, Santa Clara, CA). Purified gDNA was stored at 20°C until required.

**TR34-LAMP primer design**

The TR34 LAMP primers were designed based on 100 *cyp51A* gene related strains of *A. fumigatus,* that included sequences containing TR34 and TR46 mutant alleles as well as wildtype isolates. The sequences were downloaded from NCBI GeneBank (https://www.ncbi.nlm.nih.gov/genbank/) and were aligned by using MUSCLE algorithm (Edar, 2004) in Geneious V.10 (Kearse *et al.,* 2012). The sequence alignment was verified by identification of the tandem repeat regions. The global alignment among all strains were highly conserved, except for the 34 bp or 46 bp mutant regions. In total a 250 bp long nucleotide alignment was used for TR34 LAMP primer design. LAMP exhibits high specificity due to the use of six primers recognizing eight distinct regions: The forward and backward inner primers, FIP/BIP, play critical roles for specificity of the assay. This is because the F2 primer of FIP anneals to the F2c region in the target to initiate the LAMP reaction. The outer primers, F3/B3, are composed with fewer bases and are at a lower concentration than FIP, initiating annealing of F3 to the target in order to commence strand displacement. In addition to these four essential primers, the loop primers, LF/LB, are used to accelerate the speed of the assay.

In this study, the challenge for primer design was to select six distinct regions within a long and highly repetitive location (TR34-1 and TR34-2, Figure 2). Due to the nature of this region, the possibility of primer self-annealing and non-specific amplification is high. To overcome these difficulties, we modified the 3'end of the F2 and selected an optimal location for the F1c primer design. This is because FIP (F1c+F2) is critical for initiating the reaction, and thus may affect the specificity of the LAMP assay. The details of TR34-LAMP primer design are as follows: (1) Two outer primers (F3 and B3) and one inner primer (BIP) were automatically designed by using PrimerExplorer V5 online software (https://primerexplorer.jp). However, due to the limitation of this software for tandem repeat LAMP primer design, the other inner primer (FIP) and one loop primer (LF) were designed manually. (2) The junction between the first tandem repeat (TR34-1) and the downstream region were targeted for designing the F2 primer, as this junction is the less conserved region, and therefore the primer designed based on this region may be able to differentiate the TR34 mutant allele from other similar strains. (3) To decrease the possibility of amplifying the second repeat region (TR34-2), a mismatch was introduced on the fourth base from the 3' F2 primer terminus (Figure 2), by replacing a G with A to decrease the 3' stability of FIP. (4) The location for F1c primer design was chosen to avoid covering too much of the second repeat region (TR34 region 2), in order to decrease the possibility of self-annealing of FIP (between F1c and F2). (5) Subsequently, all these newly-developed LAMP primers were checked for secondary structure and primer dimer formation by using NUPACK (http://www.nupack.org/). Details of LAMP primers used for detection of TR34 mutation were shown in Table 1.

**Table 1. TR34-LAMP primer sequences.**

| Primer | Sequence (5' to 3') |
|---|---|
| F3 | CACCACTTCAGAGTTGTCT (SEQ ID NO: 11) |
| B3 | GTATTTTATATTCACCTACCTACCA (SEQ ID NO: 12) |
| FIP | TAATTAGGCAACTTTCAT-TCCGGATGTGTGCTGAGCCGAATAAAT (SEQ ID NO: 13) |
| BIP | ACTAAGGTGTAGTTCCAGCATACC-GTAAGTAGATCTACCTACCGTAGT (SEQ ID NO: 14) |
| LF | CGGACCGCGTGATTCAT (SEQ ID NO: 15) |

The introduced mismatch at 3' of F2 primer is shown in bold.

**LAMP reaction conditions**

To detect TR34 mutation in *A. fumigatus* using LAMP on a real-time PCR machine, the LAMP mix contained the following: 1.5 $\mu$L 10X isothermal buffer, 0.9 $\mu$L MgSO4 (stock at 100mM), 2.1 $\mu$L dNTPs (stock at 10uM each), 0.375 $\mu$L BSA (20 mg/mL), 2.4 $\mu$L Betaine (5 M), 0.6 $\mu$L of Bst 2.0 DNA Polymerase (New England Biolabs, UK) (8,000U/uL), 0.375 $\mu$L of Syto9 (20 $\mu$M stock), 1.5 $\mu$L of primer mixture (20 $\mu$M BIP/FIP, 10 $\mu$M LF, and 2.5 $\mu$M B3/F3), 3 $\mu$L various concentrations of gDNA or synthetic DNA (Integrated DNA Technologies, The Netherlands) template solution ranging from 10 to $10^6$ copies/reaction, and enough nuclease-free water to bring the volume to 15 $\mu$L. The solution was split into 5 $\mu$L reactions (triplicates) on the 96-well PCR plate and heated at 63 °C for 30 min.

**Analytical sensitivity of the LAMP assay**

Real-time LAMP sensitivity was evaluated in serial ten-fold dilutions of synthetic TR34 DNA target, ranging from 1

to $10^6$ copies per reaction. Each condition was run in triplicate and the experiments repeated twice. LAMP assays were carried out in an LC96 System (LightCycler® 96 System, Roche) and data analysis was performed using LC96 Software (version SW1.1). All experiments were performed using synthetic DNA.

**Specificity of the LAMP product**

The specificity of TR34-LAMP amplification was determined by the restriction digest of LAMP products. Based on the consensus target sequence, the restriction enzyme *MluCI* (New England Biolabs, UK) was used to digest LAMP products. The restriction digests were incubated at 37 °C for 30 min, using the CutSmart buffer provide by the manufacturer. Digested products were analysed by gel electrophoresis on a 1.2% agarose gel, in 1X TAE buffer at 120 volts for 40 min. The DNA bands were stained with SYBER safe DNA Gel Stain (Thermo Fisher Scientific, USA) and visualised by illumination with an UV lamp (366nm) using the BioSpectrum imaging system. All experiments were performed using genomic DNA from clinical isolates.

**Colorimetric LAMP method**

The WarmStart Colorimetric LAMP 2X Master Mix (New England Biolabs) contains a phenol red pH indicator which provides visual detection of the accumulation of protons during the LAMP reaction, providing a change in solution colour from pink (negative) to yellow (positive). LAMP reactions were performed in 0.2 mL PCR tubes in a 25 $\mu$L reaction volume, which contains 12.5 $\mu$L warmstart colorimetric LAMP 2X master mix, 2.5 $\mu$L TR34-LAMP primer mix (10X), 2.5 $\mu$L DNA at $10^5$ copies/uL, 7.5 $\mu$L nuclease-free water.

**Amplification and detection of clinical isolates**

Eleven clinical isolates were used to evaluate the applicability of the TR34-LAMP primers. Samples included four TR34, three TR46 and four wild types strains. Real-time experiments were performed by using the LC96 System (LightCycler® 96 System, Roche, Switzerland), while end-point experiments were conducted by an end-point PCR machine (VeritiTM Dx 96-well Fast Thermal Cycler, Thermo Fisher Scientific, USA), following by the colorimetric detection. In addition, TR46 and wild type strains were included to evaluate TR34-LAMP assay cross-reactivity.

**Mobile phone imaging protocol and image processing**

An iPhone 6s was used to capture the readout under room-light conditions. The photo was taken by phone camera with auto-focus and auto-exposure default settings. The image was subsequently processed by using open resource ImageJ software (V.1.51).

**Analytical sensitivity of the TR34 LAMP assay**

The TR34-LAMP assay was sensitive with a detection limit of 10 copies per reaction. The average time-to-positive (TTP) for the different DNA concentrations (ranging from $10^6$ to $10^1$) was 10.51 min, 12.25min, 13.67 min, 16.66 min, 17.70 min and 21.91min, respectively (Figure 4a). To evaluate the performance of the TR34-LAMP for quantification of target DNA, serial dilutions of synthetic TR34 synthetic DNA were analysed. As shown in Figure 4b, TTP versus the log of the serial dilution showed a linear regression with a correlation coefficient ($R^2$) of 0.98.

**Detection and quantification of DNA from clinical sample by TR34-LAMP assay**

The applicability of the TR34-LAMP assay for detecting TR34 mutant in clinical samples was validated by real-time and end-point amplifications. Data obtained with the real-time instrument showed positive reactions only for TR34 samples. This result suggested that there was no cross-reaction with TR46 and wildtype strains, confirming the high specificity of TR34-LAMP assay. The real-time assay yielded results within 17 min for the detection of the four clinical samples containing TR34 mutant allele. The detection times were 16.45 $\pm$ 0.31min for CL-Asp164, 15.85 $\pm$ 0.17 min for CL-Asp168, 16.03 $\pm$ 0.13 min for CL-Asp251 and 15.92 $\pm$ 0.20 min for E076 (Table 2). Obtained time-to-positive results were used to estimate the concentration of each clinical sample by using the regression formula from Figure 4 (y = -2.18x + 23.08). The obtained values were 1.10E+03, 2.07E+03, 1.71E+03 and 1.92E+03 copies per reaction respectively (Table 2).

Colorimetric end-point experiments showed that the TR34 LAMP assay could successfully be visualised by naked eye and/or using a cell phone camera after a 20 min reaction. Positive reactions, TR34 clinical samples, were observed as light yellow while negative reactions, TR46 and wild type samples, were observed as pink (Figure 5a).

Analysis via gel electrophoresis confirmed that the presence of TR34-LAMP products in the tubes which had caused the colour to change from pink to yellow, indicating that the TR34 primer set is specific for the TR34 mutation (Figure 5b and 5c).

**Specificity of the TR34 LAMP assay**

The specificity of the amplification was confirmed by restriction digestion of the amplified LAMP product. As expected, after *MluCl* restriction enzyme digestion two bands were observed (103 bp and 206 bp) (Figure 6). These results indicate that the LAMP products were specifically amplified from TR34 region within *A. fumigatus.*

**Table 2. Validation of the TR34-LAMP assay with clinical isolates.**

| Type | *A. fumigatus* detection | | | TR34 mutation detection | | |
|------|-------------------|-----------------------|----------------|--------------------|----------------|-----------------------------|
| | Clinical sample | LAMP A. *fumigatus*[a] | TTP[b] (min) | TR34-LAMP[c] | TTP[b] (min) | Estimated concentration[d] |
| **TR34** | CL-Asp164 | + | 23.19 (SD=±0.25) | + | 16.45 (SD=10.31) | 1.10E+3 |
| | CL-Asp168 | + | 23.29 (SD=±0.50) | + | 15.85 (SD=±0.17) | 2.07E+3 |
| | CL-Asp251 | + | 24.6 (SD=±1.14) | + | 16.03 (SD=±0.13) | 1.71E+3 |
| | E076 | + | 22.23 (SD=±0.28) | + | 15.92 (SD=±0.20) | 1.92E+3 |
| **TR46** | E224 | + | 22.82 (SD=±0.27) | - | - | - |
| | E357 | + | 21.32 (SD=±0.29) | - | - | - |
| | E276 | + | 22.55 (SD=±0.23) | - | - | - |
| **WT** | E409 | + | 25.82 (SD=±0.42) | - | - | - |
| | ARAF016 | + | 22.72 (SD=±0.21) | - | - | - |
| | AF293 | + | 24.98 (SD=±0.81) | - | - | - |
| | CL-ASP237 | + | 22.39 (SD=±0.14) | - | - | - |

a - LAMP-A. *fumigatus*: Result of LAMP assay by using published LAMP primers for *A. fumigatus* detection (Tang *et al.,* 2016).
b - TTP: Time to positivity (detection within 30 min timeframe).
c - TR34-LAMP: Result of TR34-LAMP assay by using clinical samples.
d - Estimated concentration: Estimation of initial concentration by linear regression formula (y = - 2.18x + 23.08)
+: Positive reaction
- : Negative reaction
SD: Standard deviation. This value was calculated from three replicates.
WT: Wild type

**Example 2** - **TR46-LAMP assay**

The method described above in Example 1 concerning the TR34-LAMP assay was applied *mutatis mutandis* in the design of a TR46-LAMP assay.
In this study, the challenge for primer design was to select six distinct regions within a long and highly repetitive location. Due to the nature of this region, the possibility of primer self-annealing and non-specific amplification is high. To overcome these difficulties, we modified the 3' end of the F2 and selected an optimal location for the F1c primer design. The mismatches were introduced between two tandem repeat, as this junction is the less conserved region, and therefore the F2 primer designed based on this region may be able to differentiate the TR46 mutant allele from other similar strains.
Details of LAMP primers used for detection of TR46 mutation were shown in Table 3.

**Table 3.** TR46-LAMP primer sequences. Mismatches are underlines.

| Primer | Sequence (5' to 3') |
|--------|---------------------|
| F3 | CACCACTTCAGAGTTGTCT (SEQ ID NO: 16) |
| B3 | GTATTTTATATTCACCTACCTACCA (SEQ ID NO: 17) |

(continued)

| Primer | Sequence (5' to 3') |
|---|---|
| FIP | TAATTAGGCAACTTTCAT-TCCGGATGTGTGCTGAGAAAAAGGAAAGTTGTCTAG (SEQ ID NO: 18) |
| BIP | ACTAAGGTGTAGTTCCAGCATACC-GTAAGTAGATCTACCTACCGTAGT (SEQ ID NO: 19) |
| LB | ACCATACACCCTAACTCATACTACGG (SEQ ID NO: 20) |

Both the TR34-LAMP assay and the TR46-LAMP assay were applied to clinical samples and environmental samples as illustrated in Table 4.

Table 4. Validation of the TR34-LAMP assay and TR46-LAMP assay with clinical and environmental samples.

| Sample | Source | Country | TR34 mutation detection | | | TR46 mutation detection | | | mutant/wildtype |
| --- | --- | --- | --- | --- | --- | --- | --- | --- | --- |
| | | | TR34-LAMP | TTP (min) | Estimated concentration | TR46-LAMP | TTP (min) | Estimated concentration | |
| C78 | environmental | Republic of Ireland | + | 12.30 (SD=±0.05) | 8.77511E+04 | - | - | - | TR34 |
| C80 | environmental | Republic of Ireland | + | 12.72 (SD=±0.14) | 5.63171E+04 | - | - | - | TR34 |
| C85 | environmental | Republic of Ireland | + | 12.99 (SD=±0.10) | 4.33419E+04 | - | - | - | TR34 |
| C86 | environmental | Republic of Ireland | + | 12.04 (SD=±0.08) | 1.15232E+05 | - | - | - | TR34 |
| C92 | environmental | Republic of Ireland | + | 12.65 (SD=±0.13) | 6.06377E+04 | - | - | - | TR34 |
| C90 | environmental | Republic of Ireland | + | 12.42 (SD=±0.06) | 7.73073E+04 | - | - | - | TR34 |
| C284 | environmental | USA | + | 12.77 (SD=±0.05) | 5.34208E+04 | - | - | - | TR34 |
| C287 | environmental | USA | + | 12.29 (SD=±0.05) | 8.86826E+04 | - | - | - | TR34 |
| C288 | environmental | USA | + | 12.52 (SD=±0.11) | 6.95602E+04 | - | - | - | TR34 |
| C133 | clinical | London | + | 13.84 (SD=±0.18) | 1.72587E+04 | - | - | - | TR34 |
| C134 | clinical | London | + | 14.52 (SD=±0.07) | 8.41705E+03 | - | - | - | TR34 |
| C143 | clinical | London | + | 13.55 (SD=±0.11) | 2.33436E+04 | - | - | - | TR34 |
| C144 | clinical | London | + | 14.90 (SD=±0.15) | 5.63497E+03 | - | - | - | TR34 |
| C160 | clinical | England | + | 14.49 (SD=±0.11) | 5.69479E+03 | - | - | - | TR34 |

| Sample | Source | Country | TR34 mutation detection | | | TR46 mutation detection | | | mutant/wildtype |
|---|---|---|---|---|---|---|---|---|---|
| | | | TR34-LAMP | TTP (min) | Estimated concentration | TR46-LAMP | TTP (min) | Estimated concentration | |
| C161 | clinical | England | + | 13.76 (SD= ±0.25) | 1.87800E+04 | - | - | - | TR34 |
| hyde42 | environmental | England | + | 14.48 (SD= ±0.20) | 8.78019E+03 | - | - | - | TR34 |
| C79 | environmental | Republic of Ireland | - | - | - | + | 13.86 (SD= ±0.20) | 2.90100E+05 | TR46 |
| C87 | environmental | Republic of Ireland | - | - | - | + | 13.98 (SD= ±0.15) | 2.55133E+05 | TR46 |
| C91 | environmental | Republic of Ireland | - | - | - | + | 14.01 (SD= ±0.14) | 2.48460E+05 | TR46 |
| C93 | environmental | Republic of Ireland | - | - | - | + | 16.03 (SD= ±0.06) | 3.15017E+04 | TR46 |
| C95 | environmental | Republic of Ireland | - | - | - | - | - | - | WT |
| C96 | environmental | Republic of Ireland | - | - | - | - | - | - | WT |
| C98 | environmental | Republic of Ireland | - | - | - | - | - | - | WT |
| C97 | environmental | Republic of Ireland | - | - | - | - | - | - | WT |
| C285 | environmental | USA | - | - | - | - | - | - | WT |
| C286 | environmental | USA | - | - | - | - | - | - | WT |
| C238 | environmental | South Africa | - | - | - | - | - | - | WT |
| C241 | environmental | India | - | - | - | - | - | - | WT |
| C242 | environmental | India | - | - | - | - | - | - | WT |
| C243 | environmental | India | - | - | - | - | - | - | WT |
| C244 | environmental | Australia | - | - | - | - | - | - | WT |
| C245 | environmental | Australia | - | - | - | - | - | - | WT |

| Sample | Source | Country | TR34 mutation detection | | | TR46 mutation detection | | | mutant/wildtype |
|---|---|---|---|---|---|---|---|---|---|
| | | | TR34-LAMP | TTP (min) | Estimated concentration | TR46-LAMP | TTP (min) | Estimated concentration | |
| C249 | environmental | South Africa | - | - | - | - | - | - | WT |

**Example 3** - **Extraction of *A. fumigatus* DNA from spiked soil samples and TR-LAMP assays**

To check soil and compost samples for presence of WT and resistant *A. fumigatus* before the start of the experiments with TR-LAMP assays, 2g of soil and compost samples were diluted with 10ml of 0.05% Tween 20 and 100 $\mu$l of supernatant was added on petri dishes with and without voriconazole. The petri dishes were incubated for two days and then colony forming units (cfu) were counted with the number of *A .fumigatus* colonies identified.

To evaluate the performance of TR-LAMP assays on *A. fumigatus* DNA extracted from soils, samples of two different soil types (New Forest soil and Verve multipurpose compost) were spiked with the series of ten-fold dilutions of conidia suspensions (range from 1 to $10^6$ copy number per TR-LAMP reaction). The conidia suspensions were based on three different isolates - DUB-48 (TR46), DUB-41 (TR34) and 47-236 (WT) and were diluted in 0.05% Tween. For every substrate and isolate a technical replicate was done and a control for both substrates with no conidia added was made.

DNeasy PowerSoil DNA Isolation Kit (Qiagen, Hilden, Germany) was utilised for extraction of genomic DNA from spiked soil samples and the method was followed according to the manufacturer's protocol with two changes. Instead of using advised vortex adaptor for homogenisation and cell lysis, TissueLyser II (Oiagen, Hilden, Germany) was used for 10 minutes at 15/s frequency. Additionally, compost samples required multiple centrifuge cycles due to the consistency of the substrate making it difficult to remove the required volume of supernatant. To test the advised step for increasing the yield of DNA, six samples out of the first $10^6$ copies per reaction set were extracted with pre-incubation at 60°C for 10 min prior to adding solution C1. All 12 samples, as well as positive and negative controls, and $H_2O$ were tested with *A. fumigatus* MAT-type PCR and then gel electrophoresis of PCR products was performed. Additionally, Qubit dsDNA High Sensitivity Assay Kit (Thermo Fisher Scientific, Massachusetts, United States) was utilised to quantify this set of extracted DNAs with Qubit 3.0 Fluorometer. There was no difference between 60°C incubation and standard conditions based on PCR and Qubit data, thus standard conditions were used due to the reduced procedure time.

For TR34- and TR46-LAMP assays, the experimental conditions were altered to ensure greater concentration of DNA for each reaction to improve the detection. Therefore, 10$\mu$l of samples were loaded into wells and nuclease-free water was removed from the recipe to allow the increase in DNA volume from 1 to 1.75 copies/$\mu$l per reaction. Based on temperature experiments done previously, TR34-LAMP was run at 62°C and TR46-LAMP was run at 63°C. The concentrations of DNA samples analysed were in $10^5$-$10^3$ copies/reaction range. $10^2$ copies/reaction set repeatedly showed no amplification during numerous tests, thus it was excluded from further experiments. The resulting data was analysed to get the values for sensitivity, specificity and positive/negative predictive values.

34 out of 41 cfu were confirmed to be WT *A. fumigatus* in the compost samples and 1 out of 2 cfu were WT *A. fumigatus* in soil samples. No presence of azole-resistant *A. fumigatus* was detected. Gel electrophoresis analysis was utilised to detect amplification of TR34, TR46 and WT samples (Figure 9), confirming the presence of *A. fumigatus* DNA in the extracted samples.

The observed limit of detection (LOD) was $10^3$ copies/reaction for TR34-LAMP with soil samples and LOD was $10^4$ copies/reaction for TR34-LAMP with compost samples and for TR46-LAMP with both soil and compost samples. An average time-to-positive (TTP) for TR34-LAMP for both sample types was 20.57 minutes at $10^5$ copies/reaction and 26.63 minutes at $10^4$ copies/reaction. An average TTP for TR46-LAMP for both sample types was 26.47 and 36.24 minutes, for $10^5$ and $10^4$ copies/reaction, respectively. No differences were detected between the substrates except for the initial presence of *A. fumigatus* WT strains being greater in compost.

Table 5. TR34 and TR46 LAMP assay validation with extracted DNA from soil or compost samples seeded with the range of known conidia concentrations. SD: standard deviation calculated from five replicates.

| Isolate | Allele | Source | Concentration after extraction (copy/reaction) | Time to positivity (mins) | No. of positive results/total no. of replicates |
|---------|--------|--------|-----------------------------------------------|---------------------------|-------------------------------------------------|
| DUB41 | TR34 | Soil | 1.00E+05 | 20.93 (SD=±0.70) | 5/5 |
| DUB41 | TR34 | Soil | 1.00E+04 | 26.42 (SD=±4.23) | 5/5 |
| DUB41 | TR34 | Soil | 1.00E+03 | 32.33 (SD= - ) | 1/5 |
| DUB41 | TR34 | Compost | 1.00E+05 | 20.22 (SD=±0.94) | 5/5 |
| DUB41 | TR34 | Compost | 1.00E+04 | 26.84 (SD=±5.44) | 5/5 |
| DUB41 | TR34 | Compost | 1.00E+03 | - | 0/5 |
| DUB48 | TR46 | Soil | 1.00E+05 | 24.85 (SD=±0.50) | 5/5 |
| DUB48 | TR46 | Soil | 1.00E+04 | 33.73 (SD=±2.14) | 5/5 |
| DUB48 | TR46 | Soil | 1.00E+03 | - | 0/5 |

(continued)

| Isolate | Allele | Source | Concentration after extraction (copy/reaction) | Time to positivity (mins) | No. of positive results/total no. of replicates |
|---|---|---|---|---|---|
| DUB48 | TR46 | Compost | 1.00E+05 | 28.08 (SD=±3.47) | 5/5 |
| DUB48 | TR46 | Compost | 1.00E+04 | 38.76 (SD=±3.81) | 4/5 |
| DUB48 | TR46 | Compost | 1.00E+03 | - | 0/5 |

Table 6 lists the results for TR34, TR46 and WT reactions for both assays and TR-LAMP results are in agreement with the isolates seeded. 70% of TR34 assay reactions resulted in the detectable amplification with TR34 mutant strain and 63.3% of TR46 assay reactions resulted in amplification with TR46 mutant strain. Based on the values from Table 7, the resulting sensitivity value for TR34-LAMP assay is 70% while specificity is 100%. The positive predictive value of the TR34-LAMP assay is 100.00% and negative predictive value is 86.96%. The value of TR46-LAMP assay sensitivity is 63.00% and specificity is 93.00%, with the positive predictive value being 90.48% and negative predictive value of 84.06%. No cross-reactivity with WT was detected for either of the assays. A TR46-LAMP detected false positives for TR34 isolate soil (35.49 minutes) and compost (34.98 minutes) samples at $10^5$ copies/reaction concentration.

Table 6. Overall TR34 and TR46 LAMP assays results for extracted soil and compost DNA panel including number of replicates, with percentages based on total replicate number. Data includes WT negatives and cross-reaction negatives between two assays and covers a concentration range of $10^5$-$10^3$ copies/reaction.

| LAMP assay | Result | TR34/DUB41 samples No. of replicates (%) (n=60) | TR46/DUB48 samples No. of replicates (%) (n=60) | WT/47-236 samples No. of replicates (n=60) | Total |
|---|---|---|---|---|---|
| TR34 | Positive | 21/30 (70.00%) | 0/30 (0%) | 0/30 | 21 |
| | Total | 30 | 30 | 30 | 90 |
| TR46 | Positive | 2/30 (6.70%) | 19/30 (63.30%) | 0/30 | 21 |
| | Total | 30 | 30 | 30 | 90 |

### Example 4 - CMOS-based Lab-on-Chip detection of TR34 and TR46 mutations in clinical and environmental samples

Hydrogen ions ($H^+$) released during PCR amplification can be detected in real-time with an on-chip array of 4,368 ion-sensitive field-effect transistors (ISFETs). The ISFET is a solid-state potentiometric sensor with a structure similar to a metal-oxide-semiconductor field-effect transistor (MOSFET), biased using a reference electrode (typically Ag/AgCl) and using the top silicon nitride ($Si_4N_3$) passivation layer as sensing material (Georgiou and Toumazou, 2009, Sensors and Actuators B: Chemical 143 (1): 211-217; Moser et al., 2016, IEEE Sensors Journal 16 (17): 6496 - 6514).

Such a system was used to demonstrate on-chip detection using the TR-LAMP assay. Figure 10 illustrates the cross-section of the ISFET in unmodified CMOS technology. The detection principle relies on the binding of protons to the top $Si_3N_4$ layer which modifies the threshold voltage of the device proportionally to the pH change in the solution. The device micromodel, shown in Figure 10, consists of capacitances at the gate of the equivalent MOSFET. The chemical contribution $V_{chem}$ varies with the pH of the solution, reflecting the production of double-stranded DNA during the amplification reaction. The formula can be expressed as:

$$DNA_n + d_{NTP} \rightarrow DNA_{n+1} + Pyrophosphate + Proton (H^+)$$

$$V_{chem} = \gamma - \alpha S_N \log[H^+]$$

where $\gamma$ is the non-chemically dependent term, $S_N$ is the Nernstian sensitivity of 59 mV/pH at ambient temperature and $\alpha$ is the deviation from this ideal sensitivity.

Hydration of the microchip surface layer in contact with the liquid causes slow temporal drift during the measurement (Georgiou and Toumazou, 2009, Sensors and Actuators B: Chemical 143 (1): 211-217; Moser et al., 2016, IEEE Sensors Journal 16 (17): 6496 - 6514), which presents challenges to extract signal associated with pH change.

In this work, we perform post-processing compensation by assuming that the drift follows an exponential relationship obtained by fitting the curves. Following the compensation, the sensor output is converted back to the linear domain to yield the traditional amplification curve, reflecting the temporal increase in DNA copies during amplification. The experimental setup for on-chip TR-LAMP assay is shown in Figure 11.

Three TR34 mutant (C287, C288, Hyde42), two TR46 mutant (C87 and C93) and two WT (C245 and C249) samples were selected for on-chip validation experiments. Non-template controls and cross-reactivity reactions (reactions containing primers mismatching the tandem repeat targets) were also analysed.

To illustrate the processing performed on the chip data, we consider the Hyde42 sample from the TR34 mutant target and show results at each processing step in Figure 12. The outputs from all exposed sensors are averaged to yield the raw output curve, which reflects both sensor drift and pH change during DNA amplification. To extract the signal, the algorithm finds the inflexion point at time T2 which corresponds to the amplification i.e. when the contribution from the pH dominates over the initial drift. Three reaction regions are then identified in the output curve using a thresholding method:

1) 0 - T1: no amplification; the output is only drift.
2) T1 - T3: amplification is happening; the output is the sum of drift and pH signal.
3) T3 - end: amplification products have saturated; the output is only drift.

Using the exponential approximation, drift is cancelled in regions 1 and 3. In region 2, we assume that the drift rate varies linearly from T1 to T3 and subtract the resulting curve from the output. Converting mV to pH based on the standard pH sensitivity of unmodified CMOS (Moser et al., 2018 IEEE Transactions on Biomedical Circuits and Systems 12 (2): 390 - 401), we yield the pH readout curve. Mapping the pH readout curve to a linear scale by exponentiating then results in the classical amplification curve which tracks the DNA copy numbers during the reaction.

We apply the algorithm to the aforementioned samples and obtain the amplification curves of Figure 12. For the TR34-LAMP specific assay, TTP were 15.44 min for C287 (TR34 mutant), 13.24 min for Hyde42 (TR34 mutant) and 15.02 min for C288 (TR34 mutant), while WT, TR46 mutant strain and non-template control remained negative in 30 min; for TR46-LAMP specific assay, TTP were 13.66 min for C87 (TR46 mutant) and 16.06 min for C93 (TR46 mutant), while WT, TR34 mutant strain and non-template control remained negative in 30 min (Figure 13). Overall, the amplification curves detected from the on-chip system measured by changes of voltage were highly comparable to the fluorescence signals received by the conventional real-time PCR machine (average TTP difference < 2min).

To ensure that the chemical signal can be used as a reliable readout of amplification, we tested pH of the pre-reaction and post-reaction mixes by using a Sentron pH meter (average pH change ≈ 0.7). In addition, the post dsDNA concentration of each pH-sensitive on-chip LAMP assay were significantly increase which suggested a successful amplification by specific TR-LAMP primers (dsDNA yield = 680 - 1025 ng/ul).

**Claims**

1. A method for detecting a tandem repeat in a nucleic acid sequence, wherein the tandem repeat comprises two or more repeat units each of which comprises at least two base pairs, wherein the method comprises

   (a) amplifying under isothermal conditions and stringent conditions a nucleic acid sequence from a sample, in a reaction mixture comprising

   (i) the nucleic acid sequence,
   (ii) a nucleic acid polymerase,
   (iii) a nucleoside triphosphate mixture,
   (iv) a forward inner primer (FIP), and
   (v) a backward inner primer (BIP),

   wherein the FIP or BIP anneals to a target region comprising one or more nucleotides from each of two repeat units, wherein the two repeat units are adjacent, wherein:

   FIP comprises a F1c region and a F2 region, wherein the F2 region of FIP anneals to the target region, wherein the F2 region of FIP comprises one or more mismatched nucleotides with respect to the target region,
   wherein the one or more mismatched nucleotides is located proximal to the 3' end of the F2 region of FIP, or
   BIP comprises a B1c region and a B2 region, wherein the B2 region of BIP anneals to the target region, wherein the B2 region of BIP comprises one or more mismatched nucleotides with respect to the target region,

wherein the one or more mismatched nucleotides is located proximal to the 3' end of the B2 region of BIP, and

(b) detecting an amplified nucleic acid sequence, wherein the detection of an amplified nucleic acid sequence indicates the presence the tandem repeat.

2. The method of claim 1 wherein the F2 region of FIP comprises 18 to 30 nucleotides, or the B2 region of BIP comprises 18 to 30 nucleotides.

3. The method of any preceding claim wherein

(a) the tandem repeat is a direct tandem repeat,
(b) the tandem repeat is less than or equal to 300 base pairs long, and/or
(c) the tandem repeat comprises two or three repeat units.

4. The method of any preceding claim wherein:

(a) each of the repeat units comprises 20 to 70 base pairs, optionally 30 to 50 base pairs,
(b) the target region comprises 10 or more nucleotides from a first repeat unit; one to 10 nucleotides from a second repeat unit; and 18 to 30 nucleotides in total,
(c) the reaction mixture further comprises a forward outer primer (F3) and/or a backward outer primer (B3), and/or
(d) the reaction mixture further comprises a forward loop primer (LF) and/or a backward loop primer (LB).

5. A method for detecting drug resistance in an organism, the method comprising detecting a tandem repeat in a nucleic acid sequence according to the method of any preceding claim.

6. A method for the diagnosis of an infectious disease or a drug resistant infection the method comprising detecting a tandem repeat in a nucleic acid sequence according to the method of any one of claims 1 to 4.

7. The method of any preceding claim wherein the method is performed extemporaneously.

8. The method of any preceding claim wherein,

(a) the nucleic acid sequence is DNA, the nucleic acid polymerase is a DNA polymerase and the nucleoside triphosphate mixture comprises dNTPs, or
(b) the nucleic acid sequence containing the tandem repeat is RNA, the method further comprises reverse transcription of the RNA to produce cDNA, the amplifying a nucleic acid sequence is amplifying the cDNA, the nucleic acid polymerase is a DNA polymerase and the nucleoside triphosphate mixture comprises dNTPs.

9. The method of any one of claims 1 to 8 wherein the sample is an environmental sample or a clinical sample.

10. The method of any one of claims 1 to 8 wherein the nucleic acid is a nucleic acid from a pathogen or a nucleic acid from a host.

11. The method of claim 10 wherein the tandem repeat is selected from the group consisting of TR34 in the *cyp51A* gene of *A. fumigatus;* TR46 in the *cyp51A* gene of *A. fumigatus;* and TR53 in the *cyp51A* gene of *A. fumigatus,* optionally wherein:

(a) the tandem repeat is TR34 in the *cyp51A* gene of *A. fumigatus* and

(i) FIP comprises

TAATTAGGCAACTTTCATTCCGGATGTGTGCTGAGCCGAATAAAT (SEQ ID NO: 1),

(ii) BIP comprises

ACTAAGGTGTAGTTCCAGCATACCGTAAGTAGATCTACCTACCGTAGT (SEQ ID NO: 2),

(iii) F3 comprises CACCACTTCAGAGTTGTCT (SEQ ID NO: 3),
(iv) B3 comprises GTATTTTATATTCACCTACCTACCA (SEQ ID NO: 4), and/or
(v) LF comprises CGGACCGCGTGATTCAT (SEQ ID NO: 5); or

(b) the tandem repeat is TR46 in the *cyp51A* gene of *A. fumigatus* and

(i) FIP comprises

TAATTAGGCAACTTTCATTCCGGATGTGTGCTGAGAAAAAGGAAAGTTGTCTAG (SEQ ID NO: 6),

(ii) BIP comprises

ACTAAGGTGTAGTTCCAGCATACCGTAAGTAGATCTACCTACCGTAGT (SEQ ID NO: 7),

(iii) F3 comprises CACCACTTCAGAGTTGTCT (SEQ ID NO: 8),
(iv) B3 comprises GTATTTTATATTCACCTACCTACCA (SEQ ID NO: 9), and/or
(v) LB comprises ACCATACACCCTAACTCATACTACGG (SEQ ID NO: 10).

12. The method of any preceding claim wherein the detecting is colorimetric detection, pH-based detection and/or detecting by one or more ion sensors, optionally
wherein the one or more ion sensors are one or more semiconductor-based ion sensors, optionally an ion-sensitive field-effect transistor (ISFET).

13. A kit comprising:

(i) a forward inner primer (FIP), and
(ii) a backward inner primer (BIP),
wherein the FIP or BIP is configured to anneal under stringent conditions to a target region comprising one or more nucleotides from each of two repeat units in a tandem repeat, wherein the two repeat units are adjacent and each of the two repeat units comprises at least two nucleotides, wherein FIP comprises a F1c region and a F2 region, wherein the F2 region of FIP is configured to anneal to the target region,
wherein the F2 region of FIP comprises one or more mismatched nucleotides with respect to the target region, wherein the one or more mismatched nucleotides is located proximal to the 3' end of the F2 region of FIP, or wherein BIP
comprises a B1c region and a B2 region, wherein the B2 region of BIP is configured to anneal to the target region, wherein the B2 region of BIP comprises one or more mismatched nucleotides with respect to the target region, wherein the one or more mismatched nucleotides is located proximal to the 3' end of the B2 region of BIP.

14. The kit of claim 13 further comprising:

(iii) a forward outer primer (F3),
(iv) a backward outer primer (B3), and
(v) a forward loop primer (LF).

15. A reaction mixture comprising the components of the kit according to claim 13 or claim 14, in a liquid medium.

**Patentansprüche**

1. Verfahren zum Nachweis einer Tandem-Repeat in einer Nukleinsäuresequenz, wobei die Tandem-Repeat zwei oder mehr Repeat-Einheiten umfasst, die jeweils mindestens zwei Basenpaare umfassen, wobei das Verfahren Folgendes umfasst:

(a) Amplifizieren einer Nukleinsäuresequenz aus einer Probe unter isothermen Bedingungen und stringenten Bedingungen in einem Reaktionsgemisch, das

(i) die Nukleinsäuresequenz,
(ii) eine Nukleinsäure-Polymerase,
(iii) ein Nukleosidtriphosphatgemisch,
(iv) einen inneren Vorwärtsprimer (Forward Inner Primer, FIP) und
(v) einen inneren Rückwärtsprimer (Backward Inner Primer, BIP)

umfasst, wobei sich der FIP oder BIP an eine Zielregion anlagert, die ein oder mehrere Nukleotide von jeder von zwei Repeat-Einheiten umfasst, wobei die beiden Repeat-Einheiten nebeneinander liegen, wobei:

FIP eine F1c-Region und eine F2-Region umfasst, wobei sich die F2-Region von FIP an die Zielregion anlagert,
wobei die F2-Region von FIP ein oder mehrere nicht passende Nukleotide in Bezug auf die Zielregion umfasst,
wobei die ein oder mehreren nicht passenden Nukleotide proximal zum 3'-Ende der F2-Region von FIP liegen, oder BIP eine B1c-Region und eine B2-Region umfasst, wobei sich die B2-Region von BIP an die Zielregion anlagert,
wobei die B2-Region von BIP ein oder mehrere nicht passende Nukleotide in Bezug auf die Zielregion umfasst,
wobei die ein oder mehreren nicht passenden Nukleotide proximal zum 3'-Ende der B2-Region von BIP liegen, und

(b) Nachweisen einer amplifizierten Nukleinsäuresequenz, wobei der Nachweis einer amplifizierten Nukleinsäuresequenz das Vorliegen der Tandem-Repeat anzeigt.

2. Verfahren nach Anspruch 1, wobei die F2-Region von FIP 18 bis 30 Nukleotide umfasst oder die B2-Region von BIP 18 bis 30 Nukleotide umfasst.

3. Verfahren nach einem vorhergehenden Anspruch, wobei

(a) es sich bei der Tandem-Repeat um eine direkte Tandem-Repeat handelt,
(b) die Tandem-Repeat eine Länge von kleiner oder gleich 300 Basenpaaren aufweist und/oder
(c) die Tandem-Repeat zwei oder drei Repeat-Einheiten umfasst.

4. Verfahren nach einem vorhergehenden Anspruch, wobei:

(a) die Repeat-Einheiten jeweils 20 bis 70 Basenpaare, gegebenenfalls 30 bis 50 Basenpaare umfassen,
(b) die Zielregion 10 oder mehr Nukleotide aus einer ersten Repeat-Einheit, ein bis 10 Nukleotide aus einer zweiten Repeat-Einheit und insgesamt 18 bis 30 Nukleotide umfasst,
(c) das Reaktionsgemisch ferner einen äußeren Vorwärtsprimer (F3) und/oder einen äußeren Rückwärtsprimer (B3) umfasst und/oder
(d) das Reaktionsgemisch ferner einen Loop-Vorwärtsprimer (LF) und/oder einen Loop-Rückwärtsprimer (LB) umfasst.

5. Verfahren zum Nachweis von Arzneistoffresistenz in einem Organismus, wobei das Verfahren Nachweisen einer Tandem-Repeat in einer Nukleinsäuresequenz gemäß dem Verfahren nach einem vorhergehenden Anspruch umfasst.

6. Verfahren zur Diagnose einer Infektionskrankheit oder einer arzneistoffresistenten Infektion, wobei das Verfahren Nachweisen einer Tandem-Repeat in einer Nukleinsäuresequenz gemäß dem Verfahren nach einem der Ansprüche 1 bis 4 umfasst.

7. Verfahren nach einem vorhergehenden Anspruch, wobei das Verfahren spontan durchgeführt wird.

8. Verfahren nach einem vorhergehenden Anspruch, wobei

(a) es sich bei der Nukleinsäuresequenz um DNA handelt, es sich bei der Nukleinsäure-Polymerase um eine DNA-Polymerase handelt und das Nukleosidtriphosphatgemisch dNTP umfasst oder
(b) es sich bei der Nukleinsäuresequenz, die die Tandem-Repeat enthält, um RNA handelt, das Verfahren ferner reverse Transkription der RNA zur Erzeugung von cDNA umfasst, das Amplifizieren einer Nukleinsäuresequenz Amplifizieren der cDNA bedeutet, es sich bei der Nukleinsäure-Polymerase um eine DNA-Polymerase handelt und das Nukleosidtriphosphatgemisch dNTP umfasst.

9. Verfahren nach einem der Ansprüche 1 bis 8, wobei es sich bei der Probe um eine Umweltprobe oder eine klinische Probe handelt.

10. Verfahren nach einem der Ansprüche 1 bis 8, wobei es sich bei der Nukleinsäure um eine Nukleinsäure aus einem Krankheitserreger oder eine Nukleinsäure aus einem Wirt handelt.

11. Verfahren nach Anspruch 10, wobei die Tandem-Repeat aus der Gruppe bestehend aus TR34 im *cyp51A*-Gen von *A. fumigatus,* TR46 im cyp51A-Gen von *A. fumigatus* und TR53 im *cyp51A*-Gen von *A. fumigatus* ausgewählt ist, gegebenenfalls
wobei:

(a) es sich bei der Tandem-Repeat um TR34 im *cyp51A*-Gen von *A. fumigatus* handelt und

(i) FIP TAATTAGGCAACTTTCATTCCGGATGTGCTGAGCCGAATAAAT (SEQ ID NO: 1) umfasst,
(ii) BIP
ACTAAGGTGTAGTTCCAGCATACCGTAAGTAGATCTACCTACCGTAGT (SEQ ID NO: 2) umfasst,
(iii) F3 CACCACTTCAGAGTTGTCT (SEQ ID NO: 3) umfasst,
(iv) B3 GTATTTTATTATTCACCTACCTACCA (SEQ ID NO: 4) umfasst und/oder
(v) LF CGGACCGCGTGATTCAT (SEQ ID NO: 5) umfasst; oder

(b) es sich bei der Tandem-Repeat um TR46 im *cyp51A-Gen* von *A. fumigatus* handelt und

(i) FIP
TAATTAGGCAACTTTCATTCCGGATGTGTGCTGAGAAAAGGAAGTTGTCTAG (SEQ ID NO: 6) umfasst,
(ii) BIP
ACTAAGGTGTAGTTCCAGCATACCGTAAGTAGATCTACCTACCGTAGT (SEQ ID NO: 7) umfasst,
(iii) F3 CACCACTTCAGAGTTGTCT (SEQ ID NO: 8) umfasst,
(iv) B3 GTATTTTATTATTCACCTACCTACCA (SEQ ID NO: 9) umfasst und/oder
(v) LB ACCATACACCCTAACTCATACTACGG (SEQ ID NO: 10) umfasst.

12. Verfahren nach einem vorhergehenden Anspruch, wobei es sich bei dem Nachweisen um einen kolorimetrischen Nachweis, Nachweis auf pH-Basis und/oder Nachweisen mit einem oder mehreren Ionensensoren handelt, gegebenenfalls
wobei es sich bei den ein oder mehreren Ionensensoren um ein oder mehrere Ionensensoren auf Halbleiterbasis, gegebenenfalls um einen ionensensitiven Feldeffekttransistor (ISFET) handelt.

13. Kit, umfassend:

(i) einen inneren Vorwärtsprimer (FIP) und
(ii) einen inneren Rückwärtsprimer (BIP),
wobei der FIP oder BIP so konfiguriert ist, dass er sich stringenten Bedingungen an eine Zielregion anlagert, die ein oder mehrere Nukleotide von jeder von zwei Repeat-Einheiten in einer Tandem-Repeat umfasst, wobei die beiden Repeat-Einheiten nebeneinander liegen und die beiden Repeat-Einheiten mindestens zwei Nukleotide umfasst, wobei FIP eine F1c-Region und eine F2-Region umfasst, wobei die F2-Region von FIP so konfiguriert ist, dass sie sich an die Zielregion anlagert,
wobei die F2-Region von FIP ein oder mehrere nicht passende Nukleotide in Bezug auf die Zielregion umfasst, wobei die ein oder mehreren nicht passenden Nukleotide proximal zum 3'-Ende der F2-Region von FIP liegen oder wobei BIP eine B1c-Region und eine B2-Region umfasst,
wobei die B2-Region von BIP so konfiguriert ist, dass sie sich an die Zielregion anlagert, wobei die B2-Region von BIP ein oder mehrere nicht passende Nukleotide in Bezug auf die Zielregion umfasst, wobei die ein oder mehreren nicht passenden Nukleotide proximal zum 3'-Ende der B2-Region von BIP liegen.

14. Kit nach Anspruch 13, ferner umfassend:

    (iii) einen äußeren Vorwärtsprimer (F3),
    (iv) einen äußeren Rückwärtsprimer (B3) und
    (v) einen Loop-Vorwärtsprimer (LF).

15. Reaktionsgemisch, umfassend die Komponenten des Kits gemäß Anspruch 13 oder Anspruch 14 in einem flüssigen Medium.

**Revendications**

1. Procédé de détection d'une répétition en tandem dans une séquence d'acide nucléique, la répétition en tandem comprenant au moins deux motifs répétitifs dont chacun comprend au moins deux paires de bases, le procédé comprenant

    (a) l'amplification, dans des conditions isothermes et des conditions stringentes, d'une séquence d'acide nucléique provenant d'un échantillon, dans un mélange réactionnel comprenant

        (i) la séquence d'acide nucléique,
        (ii) une acide nucléique polymérase,
        (iii) un mélange de nucléosides triphosphates,
        (iv) une amorce interne sens (FIP), et
        (v) une amorce interne antisens (BIP),

    dans lequel FIP ou BIP s'hybride à une région cible comprenant un ou plusieurs nucléotides de chacun de deux motifs répétitifs, les deux motifs répétitifs étant adjacents, **caractérisé en ce que** :

        FIP comprend une région F1c et une région F2, la région F2 de FIP s'hybridant à la région cible, la région F2 de FIP comprenant un ou plusieurs nucléotides mésappariés par rapport à la région cible,
        dans lequel le ou les nucléotides mésappariés sont situés à proximité de l'extrémité 3' de la région F2 de FIP,
        ou BIP comprend une région B1c et une région B2, la région B2 de BIP s'hybridant à la région cible,
        dans lequel la région B2 de BIP comprend un ou plusieurs nucléotides mésappariés par rapport à la région cible,
        dans lequel le ou les nucléotides mésappariés sont situés à proximité de l'extrémité 3' de la région B2 de BIP, et

    (b) la détection d'une séquence d'acide nucléique amplifiée, la détection d'une séquence d'acide nucléique amplifiée indiquant la présence de la répétition en tandem.

2. Procédé selon la revendication 1, dans lequel la région F2 de FIP comprend 18 à 30 nucléotides, ou la région B2 de BIP comprend 18 à 30 nucléotides.

3. Procédé selon l'une quelconque des revendications précédentes, dans lequel

    (a) la répétition en tandem est une répétition en tandem directe,
    (b) la répétition en tandem a une longueur inférieure ou égale à 300 paires de bases, et/ou
    (c) la répétition en tandem comprend deux ou trois motifs répétitifs.

4. Procédé selon l'une quelconque des revendications précédentes, dans lequel :

    (a) chacun des motifs répétitifs comprend 20 à 70 paires de bases, facultativement 30 à 50 paires de bases,
    (b) la région cible comprend au moins 10 nucléotides d'un premier motif répétitif ; un à 10 nucléotides d'une deuxième motif répétitif ; et 18 à 30 nucléotides au total,
    (c) le mélange réactionnel comprend en outre une amorce externe sens (F3) et/ou une amorce externe antisens (B3), et/ou
(d) le mélange réactionnel comprend en outre une amorce en boucle sens (LF) et/ou une amorce en boucle antisens (LB).

5. Procédé de détection d'une pharmacorésistance dans un organisme, le procédé comprenant la détection d'une répétition en tandem dans une séquence d'acide nucléique selon le procédé de l'une quelconque des revendications précédentes.

6. Procédé de diagnostic d'une maladie infectieuse ou d'une infection résistante aux médicaments, le procédé comprenant la détection d'une répétition en tandem dans une séquence d'acide nucléique selon le procédé de l'une quelconque des revendications 1 à 4.

7. Procédé selon l'une quelconque des revendications précédentes, le procédé étant réalisé de manière extemporanée.

8. Procédé selon l'une quelconque des revendications précédentes, dans lequel,

(a) la séquence d'acide nucléique est de l'ADN, l'acide nucléique polymérase est une ADN polymérase et le mélange de nucléosides triphosphates comprend des dNTP, ou
(b) la séquence d'acide nucléique contenant la répétition en tandem est de l'ARN, le procédé comprenant en outre la transcription inverse de l'ARN pour produire de l'ADNc, l'amplification d'une séquence d'acide nucléique est l'amplification de l'ADNc, l'acide nucléique polymérase est une ADN polymérase et le mélange de nucléosides triphosphates comprend des dNTP.

9. Procédé selon l'une quelconque des revendications 1 à 8, dans lequel l'échantillon est un échantillon environnemental ou un échantillon clinique.

10. Procédé selon l'une quelconque des revendications 1 à 8, dans lequel l'acide nucléique est un acide nucléique provenant d'un agent pathogène ou un acide nucléique provenant d'un hôte.

11. Procédé selon la revendication 10, dans lequel la répétition en tandem est choisie dans le groupe constitué par TR34 dans le gène *cyp51A* de A. *fumigatus* ; TR46 dans le gène *cyp51A* de A. *fumigatus* ; et TR53 dans le gène *cyp51A* de A. *fumigatus,* facultativement
dans lequel :

(a) la répétition en tandem est TR34 dans le gène *cyp51A* de A. *fumigatus* et

(i) FIP comprend TAATTAGGCAACTTTCATTCCGGATGTGTGCTGAGCCGAATAAAT (SEQ ID NO : 1),
(ii) BIP comprend ACTAAGGTGTAGTTCCAGCATACCGTAAGTAGATCTACCTACCGTAGT (SEQ ID NO : 2),
(iii) F3 comprend CACCACTTCAGAGTTGTCT (SEQ ID NO : 3),
(iv) B3 comprend GTATTTTATATTCACCTACCTACCA (SEQ ID NO : 4), et/ou
(v) LF comprend CGGACCGCGTGATTCAT (SEQ ID NO : 5) ; ou

(b) la répétition en tandem est TR46 dans le gène *cyp51A* de *A. fumigatus* et

(i) FIP comprend TAATTAGGCAACTTTCATTCCGGATGTGTGCTGAGAAAAAGGAAAGTTGTCTAG (SEQ ID NO : 6),
(ii) BIP comprend ACTAAGGTGTAGTTCCAGCATACCGTAAGTAGATCTACCTACCGTAGT (SEQ ID NO : 7),
(iii) F3 comprend CACCACTTCAGAGTTGTCT (SEQ ID NO : 8),
(iv) B3 comprend GTATTTTATATTCACCTACCTACCA (SEQ ID NO : 9), et/ou
(v) LB comprend ACCATACACCCTAACTCATACTACGG (SEQ ID NO : 10).

12. Procédé selon l'une quelconque des revendications précédentes, dans lequel la détection est une détection colorimétrique, une détection basée sur le pH et/ou une détection par un ou plusieurs capteurs d'ions, facultativement dans lequel le ou les capteurs d'ions sont un ou plusieurs capteurs d'ions à base de semi-conducteur, éventuellement un transistor à effet de champ sensible aux ions (ISFET).

**13.** Kit comprenant :

(i) une amorce interne sens (FIP), et
(ii) une amorce interne antisens (BIP),
dans lequel FIP ou BIP est configuré pour s'hybrider dans des conditions stringentes à une région cible comprenant un ou plusieurs nucléotides de chacun de deux motifs répétitifs dans une répétition en tandem, les deux motifs répétitifs étant adjacents et chacun des deux motifs répétitifs comprenant au moins deux nucléotides, FIP comprenant une région F1c et une région F2, la région F2 de FIP étant configurée pour s'hybrider à la région cible,
dans lequel la région F2 de FIP comprend un ou plusieurs nucléotides mésappariés par rapport à la région cible, dans lequel le ou les nucléotides mésappariés sont situés à proximité de l'extrémité 3' de la région F2 de FIP, ou dans lequel BIP comprend une région B1c et une région B2, la région B2 de BIP étant configurée pour s'hybrider à la région cible, la région B2 de BIP comprenant un ou plusieurs nucléotides mésappariés par rapport à la région cible, le ou les nucléotides mésappariés étant situés à proximité de l'extrémité 3' de la région B2 de BIP.

**14.** Kit selon la revendication 13 comprenant en outre :

(iii) une amorce externe sens (F3),
(iv) une amorce externe antisens (B3), et
(v) une amorce en boucle sens (LF).

**15.** Mélange réactionnel comprenant les composants du kit selon la revendication 13 ou la revendication 14, dans un milieu liquide.

Figure 1

**cyp51A**

Tandem repeat region

5′ — F3 — F2 — F1 — B1 — LB — B2 — B3 —
3′ — F3c — F2 — LF — F1c — B1c — B2c — B3c —

Forward inner primer

FIP    5′ — | F1c | F2 | — 3′

Forward primer

5′ — | F3 | — 3′

Forward loop primer

5′ — | LF | — 3′

Backward inner primer

BIP    5′ — | B1c | B2 | — 3′

Backward primer

5′ — | B3 | — 3′

Backward loop primer

5′ — | LB | — 3′

Figure 2

EP 3 802 868 B1

Figure 3

Figure 4A

Figure 4B

Figure 5

Figure 6

FIGURE 7

Figure 8

Figure 9

Figure 10

Figure 11

# Raw Chip Output

# pH readout Log scale

# H⁺ readout Linear scale

## Figure 12

Figure 13A

Figure 13B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 1809420 A
- GB 2019051597 W
- GB 1809418 A
- EP 2019065039 W

### Non-patent literature cited in the description

- **SNELDERS et al.** *Future Microbiology*, vol. 6 (3), 335-347
- **SNELDERS et al.** *Applied and Environmental Microbiology*, 2009, vol. 75 (12), 4053-4057
- **RIVERO-MENEDEZ et al.** *Journal of Fungi*, 2016, vol. 2 (3), 21
- **VERWEIJ et al.** *Clinical Infectious Diseases*, 2016, vol. 62 (3), 362-368
- **CHOWDHARY et al.** *Journal of Antimicrobial Chemotherapy*, 2013, vol. 67 (2), 362-366
- **DE PAUW et al.** *C. Clinical Infectious Diseases*, 2008, vol. 46 (12), 1813-1821
- **CUENCA-ESTRELLA et al.** *Journal of Antimicrobial Chemotherapy*, 2011, 66
- **MICELI** ; **MAERTENS**. *Seminars in Respiratory and Critical Care Medicine*, 2015, vol. 36 (5), 650-661
- **WHITE et al.** *Journal of Clinical Microbiology*, 2013, vol. 51 (5), 1510-1516
- **COSTA et al.** *Journal of Clinical Microbiology*, 2002, vol. 40 (6), 2224-2227
- **DONNELLY**. *Clinical Infectious Diseases*, 2006, vol. 42 (4), 487-489
- **KAWAZU et al.** *Journal of Clinical Microbiology*, 2004, vol. 42 (6), 2733-2741
- **LOEFFLER et al.** *The Journal of Infectious Diseases*, 2002, vol. 185 (8), 1203-1206
- **MARR** ; **LEISENRING**. *Clinical Infectious Diseases*, 2005, vol. 41 (S6), S381-S386
- **PICKERING et al.** *Journal of Clinical Microbiology*, 2005, vol. 43 (12), 5957-5962
- **RAAD et al.** *Cancer*, 2002, vol. 94 (4), 1032-1036
- **WHITE et al.** *Clinical Infectious Diseases*, 2015, vol. 61 (8), 1293-1303
- **NOTOMI et al.** *Nucleic Acids Research*, 2000, vol. 28 (12), E63
- **NAGAMINE et al.** *Molecular and Cellular Probes*, 2002, vol. 16 (3), 223-229
- **JINZHAO SONG et al.** *PLOS NEGLECTED TROPICAL DISEASES*, 2015, vol. 9 (12), e0004318
- **HAROLD SALANT et al.** *AMERICAN JOURNAL OF TROPICAL MEDICINE & HYGIENE.*, 2012, vol. 87 (5), 883-887
- *CHEMICAL ABSTRACTS*, 913830-62-3
- *CHEMICAL ABSTRACTS*, 173080-69-8
- *CHEMICAL ABSTRACTS*, 163795-75-3
- **GEORGIOU** ; **TOUMAZOU**. *Sensors and Actuators B: Chemical*, 2009, vol. 143 (1), 211-217
- **MOSER et al.** *IEEE Sensors Journal*, 2016, vol. 16 (17), 6496-6514
- **GEORGIOU** ; **TOUMAZOU**. *ensors and Actuators B: Chemical*, 2009, vol. 143 (1), 211-217
- **MOSER et al.** *IEEE Transactions on Biomedical Circuits and Systems*, 2018, vol. 12 (2), 390-401